# EUROPEAN PATENT APPLICATION

(11) **EP 3 698 758 A1**
(43) Date of publication of application: **26.08.2020**
(21) Application number: 19158258.4
(22) Date of filing: 20.02.2019
(51) Int. Cl.: A61F 2/14

(54) **DEVICE AND ASSEMBLIES FOR ORIENTED TRANSPORT, MICROSCOPIC INVESTIGATION AND ORIENTED EJECTION OF A TISSUE GRAFT OR IMPLANT**

(71) Applicant: Leibniz-Institut für Polymerforschung Dresden e.V., 01069 Dresden (DE)
(72) Inventor: BINNER, Marcus, 01069 Dresden (DE); TSURKAN, Sarah, 01217 Dresden (DE); TSURKAN, Mikhail, 01217 Dresden (DE)
(74) Representative: Maikowski & Ninnemann Patentanwälte Partnerschaft mbB

(57) **Abstract**

The invention provides a device for secure support, storage and/or transport of a tissue graft or an implant, which allows for precise microscopic investigation and/or evaluation and quality control of the tissue graft or implant during processing as well as prior to introduction into a living body. The main body of the device is a cannula or a cartridge comprising rectangular shape, preferably with rounded edges, having two opposite openings of different sizes and shapes. The first opening has a round shape with a diameter suitable for connection to a tube or a syringe nozzle. The second opening has an elliptical, round, biconvex or rectangle shape, in the latter case preferably with rounded edges that is small enough to be inserted into a small surgical incision. The transparency and preferably rounded, rectangular shape of the main body of the device permits microscopic examination of the tissue graft or implant within the device, as well as secures the tissue graft or implant position during delivery and implantation. The elliptical, round, biconvex or preferably rounded rectangle inner shape of the second opening ensures the correct positioning of the tissue graft or implant during surgical implantation, thereby preventing issues such as loss of the tissue graft or implant orientation. The invention further provides assemblies for loading, storing and/or transporting a tissue graft or implant comprising the device according to the invention. The invention further provides a washing assembly and a method for preparing a tissue graft or implant.

## Description

### Field of the invention

The invention provides a device for secure support and/or transport of a tissue graft or an implant, which allows for precise microscopic investigation and/or evaluation and quality control of the tissue graft or implant during processing as well as prior to introduction into a living body. The main body of the device is a cannula or a cartridge comprising rectangular shape, preferably with rounded edges, having two opposite openings of different sizes and shapes. The first opening has a round shape with a diameter suitable for connection to a tube or a syringe nozzle. The second opening has an elliptical, round, biconvex or rectangle shape, in the latter case preferably with rounded edges that is small enough to be inserted into a small surgical incision. The transparency and preferably rounded, rectangular shape of the main body of the device permits microscopic examination of the tissue graft or implant within the device, as well as secures the tissue graft or implant position during delivery and implantation. The elliptical, round, biconvex or preferably rounded rectangle inner shape of the second opening ensures the correct positioning of the tissue graft or implant during surgical implantation, thereby preventing issues such as loss of the tissue graft or implant orientation. The invention further provides assemblies for loading, storing and/or transporting a tissue graft or implant comprising the device according to the invention. The invention further provides a washing assembly and a method for preparing a tissue graft or implant.

### Background of the invention

The human cornea is one of the most transplanted tissues. Introduced more than 100 years ago, penetrating keratoplasty (PK) is a procedure for transplantation of the whole cornea including all of its layers (epithelium, Bowman's membrane, stroma, Descemet's membrane and endothelial cell layer) [1, 2]. This surgical technique has initial success of about 80-90%, but patient recovery is slow and vision is completely restored only rarely because of the geometrical differences in the donor and the recipient eyes. Moreover, long-term monitoring of patients revealed a high probability of the graft failure with more than 50% of patients requiring a second transplant within 10 years of the procedure [1].

Disorders of the corneal endothelium do not require a whole cornea transplantation. Hence, endothelial keratoplasty (EK) methods are currently the most widely used [2]. Correspondingly, endothelial keratoplasty techniques, which include the transplantation of only a thin corneal stroma layer in combination with the Descemet's membrane and the endothelial cell layer (Descemet's stripping endothelial keratoplasty [DSEK] or Descemet's stripping automated endothelial keratoplasty [DSAEK]) have been developed as alternative to PK [3]. These techniques are more comfortable in surgical procedure and significantly better in visual recovery and corresponding patient satisfaction in comparison with PK treatment. Nevertheless, these techniques still embrace a high presence of recovered vision imperfection and significant occurrence of the long-term graft rejection (15-25%) caused by the stromal part of the implant [4, 5].

In the recent decade, new endothelial keratoplasty techniques (Descemet's membrane endothelial keratoplasty [DMEK] and Pre Descemet's endothelial keratoplasty [PDEK] with a slightly thicker graft), which include the transplantation of only the Descemet's membrane and the endothelial cell layer, were developed [6, 7]. The advantages of these techniques include very fast patient retrieval with nearly complete vision recovery within a few weeks after surgery. Because of the stroma absence, there is almost no imperfection in vision of the recipient's eye independently on the geometrical differences between the eye of the donor and the recipient. In other words, most likely only DMEK and PDEK can provide nearly complete vision recovery within few weeks after operation, while patients of traditional DSEK/DSAEK and PK will never fully recover to the complete vision due to the geometry inefficiency of these implants. Additionally, DMEK/PDEK procedures result in a very low graft rejection rate (<5%) in comparison with both PK and DSEK/DSAEK surgeries [3, 5, 8-12]. DMEK and PDEK are currently considered to be the most promising keratoplasty procedures and rise rapidly worldwide as the keratoplasty technique of choice.

Recently established ultrathin (ut-) DSEK/DSAEK or nanothin (nt-) DSEK/DSAEK techniques have been reported to have similar benefits, as their thickness could be only a few micrometers larger than the DMEK and PDEK implants [13]. Similar to DMEK/PDEK tissues, the transplantation of ut-/nt-DSEK/DSAEK implants/grafts significantly improves post-operative visual outcomes with shorter recovery times and lowers graft rejection rates compared to traditional DSEK/DSAEK and PK [14]. Despite these benefits, DMEK/PDEK as well as ut-/nt-DSEK/DSAEK technologies have created challenges in graft handling due to extreme graft fragility (for both tissue integrity and cell quantity) which confines the graft processing and delivery. The originally proposed delivery in a beaker filled with media often resulted in the grafts impairment [13]. The grafts can be easily damaged by simple touching with metal instruments during preparation or surgical operation. Current state of the art methods suggest the graft handling by aqueous flow ("no touch") or on instrument manipulation at the periphery of the graft where the damage is not so crucial for later sight recovery [15-17]. The grafts fragility required additional quality control by the eye/tissue banks after the preparation as well as by the surgeon during the surgery. Nevertheless, the benefits of their application resulted in the steady growth of the operations, which in some hospitals already exceed 50% of the cornea transplantation cases [18].

Success of transplantation surgery strongly depends on the epidemiology and quality (tissue integrity and cell quantity) of the graft/implant, which is delivered from a donor to the patient [18]. The epidemiology of the transplants is fully controlled by tissue banks, which retract more than 25% of implants due to bacterial and fungal contamination as well as some type of cancers and infection diseases of the donors [18]. The quality control of the transplants in terms of tissue integrity and cell quantity can be theoretically performed in both tissue/eye banks and the operating room (OR). However, current medical standards [19] require this quality assurance to be performed by the tissue/eye banks via specular microscopy and slit-lamp examination. Nevertheless, because of the high probability of transport-associated damage with current transport solutions, there is growing demand to evaluate the implant in the OR right prior the implantation in order to prevent the implant miscarriage [20].

A crucial feature of the thin EK tissues is the correct graft orientation (apical-basal polarity ['top and bottom'] of corneal endothelial cell layer) to ensure the graft function. To ensure the right graft orientation is a great issue for surgeons [20], which in the case of the loss of the graft orientation have to return the implant back to the eye bank due to incapability to determine it in the OR. The damage related to transportation and surgery-associated manipulation of ut-/nt-DSEK/DSAEK and DMEK/PDEK implants as well as the loss of implant orientation during the operation often requires repeating the transplantation process. In order to address these challenges, new ways of graft processing, evaluation, delivery and transplantation have to be developed.
DMEK could be prepared in the OR by surgeons. This neglects the transportation issues, but significantly increases the operation times as well as lowers the quality of the implants because the OR is usually not designed for such procedures. The growing popularity of ultrathin EK grafts/implant procedures in combination with their preparation difficulties in the OR requires tissue/eye banks (e.g. for DMEK/PDEK and ut-/nt-DSEK/DSAEK) to provide improved, user-friendly solutions for tissue transport as well as to assist surgeons during implantation [21].

The "pre-stripping" approach in which the corneal endothelial lamella is fully cut, but only partially striped (removed) from the corneal stroma is an alternative processing and delivery technology for DMEK tissues [22, 23]. In this safe method for the delivery of DMEK graft into the OR compared to the delivery of a "pure" DMEK graft, the tissue graft or implant is provided in a liquid medium-filled beaker. The term 'medium' in this context means a liquid nutrition combination of necessary components for the maintenance of cell viability during implant storage, evaluation and/or transport. However, the main drawback of this technique is that the final graft striping from the corneal stroma has to be done by the surgeon right in the OR. With this, the probability of graft failure increases due to improper (non-standardized) tissue handling as well as loss of graft orientation during the surgery.

To tackle this problems, tissue banks prepare "pre-stripped & pre-loaded" DMEK and DSAEK tissues which are delivered to the OR within special assemblies of devices, of which the main part is a tissue/graft implant injector such as Straiko Modified Jones Tube [24, 25], intraocular lens cartridge [26] or Geuder DMEK injector [27]. Utilization of these "pre-stripped & pre-loaded" tissues/graft implants reduces both time in the OR and variety of complications that appears if tissue preparation fails in the OR during surgery.

However, "pre-stripped & pre-loaded" tissues/graft implants require an additional level of quality assurance in OR as the integrity of the implant as well as the cell properties should be evaluated right prior the implantation in order to prevent the transplantation miscarriage. The current solutions for the "pre-stripped & pre-loaded" tissue preparation implements the post-processing evaluation of the implant/graft by the tissue/eye banks via precision microscopy methods such as specular microscopy, slit-lamp examination, light microscopy, and/or optical coherence tomography. Currently, this evaluation requires a significant time (up to 45 minutes) at the eye bank quality control facility due to the difficulties to acquire a good microscopic images of the cornea tissues inside of the current delivery devices [28]. The application of such methods in the OR right prior the implantation is not available at this moment as the current containers and/or assemblies for pre-stripped preloaded tissue transport and implantation are not designed for the precision microscopy methods and due to the round shape of the tissue/graft implant injectors, which causes various light reflection artifacts. Therefore, there is still a strong necessity in the creation of a transport device, which would allow for both secure graft implant injection into the living body as well as precision microscopy for graft visualization. Preferably, this device should also serve as a tissue graft/implant injector, which would allow the minimum manipulation of the tissue prior the implantation in order to tackle the fragility of such tissue grafts/implants.

Recently, a new technology of corneal endothelial lamellae processing was introduced commonly named "endothelium in" or "tri-folded" DMEK. In this technology, the implant is manually folded into an 'envelope' shape during stripping so that the endothelial cell layer is facing itself, which is opposite to the natural implant rolling ("endothelium out") [26]. Remarkably, this process does not induce excessive corneal endothelial cell damage [29]. If transferred as a free-floating tissue to a solution, the implant will unroll to reach its natural "endothelium out" folding within a few minutes. Nevertheless, a few minutes is enough to insert the "endothelium in"/"tri-folded" DMEK implant envelope into an injector which prevents implant "opening" because the wall of the injector will hold the implant into folded "endothelium in"/"tri-folded" position. The insertion of such an implant into the eye of a patient results in the opening of the implant into its natural shape, thus allowing fast positioning and attachment onto the patient's corneal endothelium without much of implant manipulation. The "endothelium in"/"tri-folded" corneal lamella implantation technology has found to be advantages over the traditional "endothelium out" method as it significantly decrease the operation time and the implant manipulation in the OR [29]. It also does not require advance training of eye surgeons as the implant opens itself thus decreasing possible operation mistakes. On the other hand, the position of the implant in the injector is critical for the success of the operation and requires persist control of the implant orientation in the injector by the eye bank technician. The wrong orientation of the implant during operation can result in its termination and implant lost. The orientation control during "endothelium in"/"tri-folded" corneal lamella implantation procedure requires application of "pull through" techniques, when the implant is removed from the injector into the eye by a surgical micro-forceps similarly to DSEK/DSAEK operations [26]. Use of surgical micro-forceps for "pull though" of DSEK/DSAEK implants is acceptable due to the size and the thickness of the implant but can result in a damage of the much thinner "endothelium in"/"tri-folded" DMEK. In principal, "endothelium in"/"tri-folded" DMEK tissues can be injected as done for convenient "endothelium out" DMEK implants. However, current symmetric/round DMEK injectors (e.g. Geuder DMEK injector or Straiko Modified Jones Tube) do not allow to control and maintain the "endothelium in"/"tri-folded" DMEK orientation and some new solutions are urgently needed.

Independently on the applied technologies, implant storage, evaluation/quality control, transport and injection requires the use of several protocols by government regulations. First, the implant should be stored and transported in an approved liquid medium with defined volume, which is much larger than volume of the injector. Second, the cell density/quality of the implant must be determined after the last manipulation step, which is loading the tissue into the storage/transport device. Third, the implant must be washed with balanced salt solution (BSS) prior the injection because components of the liquid transport medium should not get into the eye of the patient. The term 'BSS' comprises buffered aqueous saline solutions such as Hank's BSS, Earle's BSS, Tyrode's salt solution, Alsever's salt solution, phosphate-buffered saline (PBS), Tris-buffered saline (TBS), Puck's salt solution, Gey's salt solution, Ringer's salt solution, Simm's salt solution and related buffered saline solutions. These regulations resulted in the use of injectors as a part of assembly that includes a container with liquid medium of corresponding volume that can hold the injector in place for the transportation and storage. Unfortunately, these injectors are not designed for the precision microscopy/tissue quality assurance methods due to their round shape, which causes various light reflection artifacts after the final assembly inside the storage/transport container. Moreover, testing of e.g. fungal infections is strongly recommended by sample drawing of the surrounding liquid medium right in the proximity of the tissue graft or implant to prevent any false-negative results [30]. This means, that the liquid medium should be drawn right inside the device loaded with the tissue graft or implant, which is already positioned inside the assemblies of devices used for transportation and/or storage. This is currently not possible, for example when using the Geuder DMEK injector with two closing caps [30]. Beneficial in this case would also be the use of a storage/transport container with two openings, where one opening is suited for insertion of the injector holder including the injector and the second opening is suited for direct aspiration of the liquid medium from the inside of the device. This prevents any additional manipulation or re-positioning of the injector within the assemblies of devices used for transportation and/or storage. Moreover, protocols and devices are needed, which allow rinsing the implant with BSS prior the injection into the living body without damaging or excluding the implant from the injector. Therefore, innovation in the injector development must include the description of the storage/transport container as well as the washing and staining accessories/protocols for the implant in the injector.

### Summary of the invention

The object of the present invention is to eliminate the disadvantages from the state of the art technology, therefore minimizing tissue graft or implant loss due to damaging the cell material by handling the tissue graft or implant or due to loss of orientation of the tissue graft or implant.

The object of the invention is solved by providing a device, an assembly for tissue graft or implant loading into the device, an assembly for transporting and/or storing the tissue graft or implant inside the device which is located inside a transport container, a washing and staining assembly and a method for preparing a tissue graft or implant as described in the following.

In one embodiment, the invention provides a device for secure support and/or storage and/or transport of a tissue graft or an implant, which allows for precise microscopic investigation and/or evaluation/quality control of the tissue graft or implant during processing as well as prior to introduction into a living body. The main body of the device is a cannula or a cartridge comprising rectangular shape, preferably with rounded edges, having two opposite openings of different sizes and shapes. The first opening has a round shape with a diameter suitable for connection to a tube or a syringe nozzle. The second opening has an elliptical, round, biconvex or rectangle shape, in the latter case preferably with rounded edges that is small enough to be inserted into a small surgical incision. The shape of the inner hollow compartment of the device, especially for the second opening, can (but must not) be different from the outer shape of the device. The transparency and preferably rounded, rectangular shape of the main body of the device permits microscopic examination of the tissue graft or implant within the device, as well as secures the tissue graft or implant position during delivery and implantation. The elliptical, round, biconvex or preferably rounded rectangle inner shape of the second opening ensures the correct positioning of the tissue graft or implant during surgical implantation, thereby preventing issues such as loss of the tissue graft or implant orientation. The elliptical, round, biconvex or rounded rectangle outer shape of the second opening also decreases tissue tension and corresponding damage to the surgical incision as it ensures a well-fitting wound closure while the device is situated in the eye.

The invention further provides assemblies for loading, storage and/or transportation of a tissue graft or implant comprising the device according to the invention. The invention further provides a washing assembly and a method for preparing a tissue graft or implant.

In a preferred embodiment of the invention, the assembly further comprises a device holder. Advantageously, this embodiment enables secure transport of the device with the tissue graft or implant, keeping the orientation of the tissue graft or implant stable. Furthermore, the assembly is suitable for the application of microscopy techniques of the tissue graft or implant, while the tissue graft or implant is located inside the device and inside the device holder, which is placed inside a storage/transport container. Furthermore, the assembly comprising the device holder is suitable for sterile packaging, evaluation and quality control as well as transportation and/or storage of the tissue graft or implant.

Furthermore, the invention provides a washing assembly for washing and staining the tissue graft or implant inside the device according to the invention. The washing assembly according to the invention is easy to use and unnecessary manipulation of the tissue graft or implant is omitted.

Additionally, a method for preparing a tissue graft or implant using the device according to the invention is provided. The method allows loading a tissue graft or implant inside the device according to the invention, transporting and/or storing the tissue graft or implant inside the device, applying evaluation and quality control techniques and washing and staining of the tissue graft or implant before ejection of the tissue graft or implant out of the device.

Especially ultrathin tissue grafts or implants for ophthalmological interventions like DMEK, PDEK and ultrathin-/nanothin-DSEK/DSAEK or related grafts or implants (e.g. "endothelium in"/"tri-folded" corneal grafts or implants) benefit from the invention and are therefore preferably used with the present invention.

The present invention enables quality control of the tissue graft or implant in the eye bank as well as in an OR via microscopy methods, whereby manipulation of the tissue graft or implant is minimized. Furthermore, a control of the tissue graft or implant orientation during the preparation, evaluation, storage, transportation and ejection is enabled, which makes the present invention especially suitable for both "endothelium in"/"tri-folded" and "endothelium out" corneal tissue grafts or implants.

The present invention provides a device and assemblies suitable for tissue graft or implant storage and/or transport and furthermore for injecting a tissue graft or implant into a living body that facilitates microscopic evaluating and/or examination of the tissue graft or implant within the device, which can be located within the assemblies. More specifically, the present invention relates to corneal tissue sample storage, handling, transport, viewing and/or evaluating after preparation/during processing and/or prior to transplantation into a living body, in particular for ophthalmological interventions.

### Detailed description of the invention

The present invention provides an optical transparent device which allows fast and precise microscopic investigation for quality control and ensures the right orientation ('top and bottom') of a tissue graft or transplant and/or an implant during the transport and/or storage and/or implantation of these tissue grafts or implants during processing as well as right prior the introduction into a living body. The device according to the invention is suitable for secure support of tissue grafts or implants during evaluation, storage and/or transport. Suitable tissue grafts or implants are for example DMEK, PDEK and ultrathin-/nanothin-DSEK/DSAEK grafts, or related grafts or implants. Especially, for handling "endothelium in"/"tri-folded" corneal grafts or implants, the present invention has clear benefits.

The engineering features of the device allow for both microscopy analysis as well as secured orientation of the implant during preparation, evaluation, storage, transportation and implantation, which simplifies the work of the technical controlling staff during implant processing and preparation as well as for the surgeons during the implantation process.

### Device

In a first aspect, the present invention provides a hollow device for secure support during storage and/or transport of a tissue graft or implant, comprising:
- a first opening,
- a main body,
- a taper area, and
- a second opening,
wherein the first opening has a round shape and a funnel-like design configured to connect with a tube or a syringe and the main body is transparent and has a rectangle shape and the taper area is transparent and has a an elliptical, round, biconvex or rectangle shape and wherein the main body comprises at least two flat and parallel opposite sites. Funnel-like design means that the diameter of the opening increases slightly towards the edge. This configuration allows precise evaluation and quality control of the tissue graft or implant inside the device. Furthermore, the orientation of the tissue graft or implant during evaluation, storage and/or transportation remains unchanged inside the device. The device is transparent for microscopy purposes and provided in accordance with the regulations for the use in clinical practice.

The first opening has a round shape with an inner diameter suitable for connection with a tube and/or a syringe nozzle which provides a smooth transition between the rounded rectangular shape of the device body as well as the connection to the round shape of the syringe and/or a tube. In one embodiment of the invention, the first opening has an inner diameter suitable to connect a tube and/or a syringe nozzle with a Luer Slip or a Luer Lock connector (according to ISO 80369). In a preferred embodiment, the inner diameter of the first opening is in the range of 3 mm to 6 mm, preferably in the range of 4 mm to 5 mm. In a preferred embodiment of the invention, the dimensions of the first opening of the device are sufficient for gentle tissue graft or implant uptake due to its funnel-like design.

The main body of the device is a cannula or a cartridge and enables stable implant positioning during the microscopic evaluation, storage and transportation. The main body comprises preferably at least two flat and parallel opposite sides. In a preferred embodiment of the invention, the main body has a rounded rectangular shape. "Rounded rectangular shape" means a rectangular shape with rounded edges. It is further preferred that the main body consists of a transparent material.

The taper area connects the main body of the device and the second opening and is also the area where the tissue graft is positioned right before the injection. The taper area has an elliptical, round, biconvex or rectangle shape, in the latter case preferably with rounded edges. In one embodiment of the invention, the taper area comprises at least two flat opposite sites analogous to the main body. In one embodiment of the invention, the inner wall and the outer wall of the taper area have the same shape. In another embodiment of the invention, the outer wall of the taper area can have a different shape than the inner wall.

In a preferred embodiment of the invention, the taper area is composed of a transparent material and has a rectangular shape with rounded edges. The transparency, the rounded rectangle shape together with the flat opposite sides of the main body of the device and the taper area allow the microscopic examination of the tissue graft or implant within the device as well as securing the tissue graft or implant orientation during the whole delivery and implantation process. Using state of the art devices for transport of tissue grafts or implants, microscopic examination (such as specular microscopy, slit-lamp examination, light microscopy and optical coherence tomography) is difficult due to various light reflection artifacts caused by the (purely round) shape of these devices. Therefore, the device according to the invention advantageously comprises flat opposite sides and a rounded rectangle shape, where light reflection artifacts as well as tissue graft or implant tension are minimized. Therefore, the area of the main body is particularly suitable for microscopic examination and in case the taper area has a rounded rectangular shape as well, also the taper area provides the above mentioned advantageous for microscopic examination.

Preferably, for the taper area the distance between top and bottom wall of its inner hollow compartment as well as its outer shape is smaller compared to the main body. Advantageously, this configuration prevents an unwanted release of the tissue graft or implant through the second opening during evaluation, storage and/or transport, while the tissue graft or implant is located within the main body of the device. Second, this configuration gives the option to seal only the first opening of the device with a cap for tissue graft or implant transport, while the second opening remains open. The tapering of the main body towards the taper area and/or the second opening of the device is sufficient enough to ensure stable tissue graft or implant positioning within the main body of the device without slipping out, even with untypically high shear forces during evaluation, storage and/or transportation. This allows free oxygen/nutrient exchange between the liquid inside the device and the surrounding liquid (e.g. nutrition medium) during transportation. The term 'medium' in this context means a liquid nutrition combination of necessary components for the maintenance of cell viability during implant storage, evaluation and/or transport. Any medium used in the clinic practice/current state of the art could be used as by the definition the proposed device does not interact with or modifies the medium as well as the medium does not change the properties of the device. Furthermore, this gives the possibility for sample drawing of surrounding solution (e.g. nutrition medium) right in the proximity of the tissue graft or implant while being located inside the device, e.g. for fungal infection tests with a decreased probability of false-negative results in comparison to state of the art devices [30].

The device comprises a second opening opposite to the first opening, wherein both openings have similar sizes and shapes, or preferably have different sizes and shapes.

The second opening side is used for the ejection of the tissue graft or implant. In a preferred embodiment, the second opening has an elliptical, round, biconvex or rounded rectangle shape, which is preferably small enough to be inserted into a small surgical incision (2.4 mm to 3.0 mm incision width). In one embodiment of the invention, the outer wall of the second opening has the same shape than the inner wall. In another embodiment, the outer wall of the second opening can have a different shape than the inner wall.

The elliptical, round, biconvex or rounded rectangle inner shape of the second opening allows for maintaining the orientation of the tissue graft or implant during the ejection of the tissue graft or implant out of the device during a surgical implantation, thus preventing issues with the loss of the tissue graft or implant orientation.

Moreover, in one embodiment of the present invention, the second opening comprises preferably a small chamfered shape opening, which is suitable for implant surgical insertion and incision wound sealing. The engineered chamfered shape of the second opening guaranties first, an easy insertion of the device into the surgical incision and second, provides the implant support during the implantation. For insertion of the tissue graft or implant, the longer wing of the chamfered part preferably faces the bottom (positioned towards the posterior eye chamber), ensuring the implant injection into the anterior chamber of the eye.

The elliptical, round, biconvex or rounded rectangle shape of the taper area and the second opening also decreases the tissue tension and corresponding damage during the device insertion because of better wound sealing of small surgical incisions (2.4 mm to 3.0 mm incision width).

In one embodiment of the invention, the inner hollow of the compartments (main body, taper area and second opening) of the device is the same as the outer shape of these compartments of the device. In another embodiment, the shape of the inner hollow compartments of the taper area and second opening of the device can be different from the outer shape of these compartments of the device. This has the advantage, that the best performance for both stable orientation and feasible microscopic investigation of the tissue inside the device as well as optimal functioning during the operation process (such as wound closure of the surgical incision while the device is inserted) is ensured. Therefore, in one embodiment of the present invention, the inner shape of the taper area and the second opening is a rounded rectangle while the outer shape is elliptical.

The individual parts of the device according to the invention can have different dimensions. In one embodiment, the diameter of the inner hollow compartment of the first opening is between 2 mm and 10 mm, preferably between 3 and 8 mm, more preferably between 3 and 6 mm, most preferably between 4 mm and 5 mm.

In one embodiment of the invention, the distance between top wall and bottom wall (which are flat and parallel to each other) of the inner hollow compartment of the main body is between 1 mm and 5 mm, preferably between 1.5 mm and 4 mm, more preferably between 2 mm and 3 mm.

According to the invention, the distance between top wall and bottom wall of the inner hollow compartment of the taper area is between 0.5 mm and 4 mm, preferably between 0.8 mm and 2 mm, more preferably between 1 mm and 2 mm.

The distance between top wall and bottom wall of the inner hollow compartment of the second opening is in one embodiment equivalent to the distance between top wall and bottom wall of the inner hollow compartment of the taper area. In another embodiment of the invention, the distance between top wall and bottom wall of the inner hollow compartment of the second opening is smallerthan the distance between top wall and bottom wall of the inner hollow compartment of the taper area.

According to the invention, the main body has a constant wall thickness. The wall thickness is between 0.1 mm and 1.0 mm, preferably between 0.1 mm and 0.8 mm, more preferably between 0.1 mm and 0.6 mm or between 0.2 mm and 0.4 mm. Most preferably, the wall thickness is 0.3 mm.

The wall thickness of the taper area and the second opening can vary and depends on the overall shape and/or the difference between the shape of the outer and inner wall of the taper area and the second opening. In case the second opening has the same shape as the taper area, the wall thickness is constant. In this event, the wall thickness is 0.1 mm and 1.0 mm, preferably between 0.1 mm and 0.8 mm, more preferably between 0.1 mm and 0.6 mm or between 0.2 mm and 0.4 mm. Most preferably, the wall thickness is 0.25 mm.

In a further embodiment, the device has a total length between 20 mm and 100 mm, preferably between 20 mm and 75 mm, more preferably between 25 mm and 50 mm, most preferably between 30 mm and 40 mm.

The shape and dimensions of the device according to the invention also allow for the insertion of tissue grafts or implants in an oriented manner because the inner shape of the device prevents the tissue grafts or implants from turning inside the device even during the forward or backward movement along the inner hollow compartment of the device, (e.g. during graft re-positioning for implantation after the transport, which is described later), thus assuring the stability of the tissue graft or implant orientation. This is especially beneficial for the application of "endothelium in"/"tri-folded" DMEK implants as it allows for maintaining the implant orientation during the direct injection into the living body, without the need of surgical forceps as during conventional "pull through" technique.

Conventional DMEK injectors are typically made from borate glass because the device surfaces are therefore preferably smooth to prevent implant damage during injection. Nevertheless, the use of glass has some significant disadvantages because glass is very fragile and can break easily, potentially not only during tissue transportation but also during surgery. Further preferably, the injector tip is also small in order to fit into the small surgical incisions (2.4 mm to 3.0 mm incision width), which requires the use of injector with thin glass walls. This aggravates the possibility of device damage/breakage.

Nevertheless, in one embodiment of the invention, the device is made from glass, preferably borate glass.

To overcome the above mentioned disadvantages of devices consisting of glass, in a more preferred embodiment of the invention, the device is made of transparent plastics which is tougher than glass, but shows similar transparency. This type of glass is in the following called "glass-like plastic". Moreover, plastics can be produced with much thinner wall thickness than any glass (e.g. via injection molding or 3D printing) without the risk of breakage during the operation procedure, opening new possibilities for even smaller incisions on the patient's eye than possible with conventional devices. The transparency of the device enables the application of evaluation and quality control of the tissue implant or graft by precision microscopy methods such as specular microscopy, slit-lamp examination, light microscopy and optical coherence tomography. In a most preferred embodiment, the plastics used for preparing the device according to the invention is highly transparent. According to the invention, the refractive index (rᵢ) is in the range of rᵢ = 1.30 to rᵢ = 1.71, preferably rᵢ = 1.30 to rᵢ = 1.65, most preferably 1.30 to rᵢ = 1.60, which is similar to the rᵢ of balanced salt solution (BSS, around 1.33 to 1.34 at 20 °C and around 600 nm), which can be used for the loading, evaluation and/or storage of tissue transplants within the device. The term 'BSS' comprises buffered aqueous saline solutions such as Hank's BSS, Earle's BSS, Tyrode's salt solution, Alsever's salt solution, phosphate-buffered saline (PBS), Tris-buffered saline (TBS), Puck's salt solution, Gey's salt solution, Ringer's salt solution, Simm's salt solution and related buffered saline solutions. It is also possible to use plastic compositions, such as combinations, mixtures, blends or copolymers of two or more plastics to manufacture the device of the invention. Plastic compositions, which are suitable for the device of the present invention because they are fitting under these requirements, are listed in Table 1. In one embodiment of the invention, the device is made of a material listed in Table 1. In a preferred embodiment, the device is made of polyacrylates, polycarbonates or, polystyrenes.

Moreover, there are specific coatings available in order to make the surface of the plastic even smother than borate glass. Both hydrophobic and hydrophilic coatings could be used to smooth the surface.

In one embodiment of the present invention, the inner surface of the device comprises a coating, wherein the coating can be a hydrophobic or a hydrophilic coating.

Hydrophobic coatings smooth the surface and create a strong surface tension, preventing the implant to "touch" the surface while being in solution, which is perfect for convenient "endothelium out" DMEK and PDEK tissue grafts or implants. Hydrophobic coatings suitable for the device of the present invention are listed in Table 2. In one embodiment of the present invention, the inner surface of the device comprises a hydrophobic coating, coating blends, coating mixtures or other combinations of hydrophobic coatings selected from Table 2.

In a preferred embodiment of the present invention, the inner surface of the device comprises a hydrophobic coating, selected from coatings comprising acrylate, organo-siloxane, silane, epoxy, a polymer, Molybdenum disulfide, Molybdenum disulfide/graphite, Tungsten disulfide or graphite, preferably selected from coatings comprising a polymer, organo-siloxane, silane, acrylate or epoxy.

Hydrophilic coatings smooth the surface and additionally act as lubricants allowing easier sliding of the implant against the surface. Thus, hydrophilic coatings are best for implants which push against the surface of the device's inner compartment such as "endothelium in"/"tri-folded" DMEK and ultrathin-/nanothin-DSEK/DSAEK tissue grafts or implants. Hydrophilic coatings suitable for the device of the present invention are listed in Table 3. In one embodiment of the present invention, the inner surface of the device comprises a hydrophilic coating, coating blends, coating mixtures or other combinations of hydrophilic coatings selected from Table 3.

In a preferred embodiment of the present invention, the inner surface of the device comprises a hydrophilic coating, selected from coatings comprising any hydrophilic polymer/hydrogel, preferably selected from coatings comprising poly(ethylene glycol), poly(acrylate), poly(methacrylate) or a UV/photo-active polymer.

A third way to modify the contact angle between a liquid and a solid surface is the use of millimeter-scaled patterning as well as micro- or nanostructured surface patterns or combinations thereof (e.g. nano-microstructuring [31]). Millimeter-scaled patterning, if aligned in the direction of the graft injection/ejection allows the control of the graft orientation, while nanoscale surface patterning is known to increase the hydrophobicity of surfaces [31, 32]. The use of micropatterning is considered to be the best option as it combines the benefits of millimeter- and nanometer-scaled patterning and can be produced by injection molding. The combination of micro- and nanostructured patterning allow from one side to keep the clear microscopic visibility of the implant inside the device for quality control, and from another side to bring the interaction of the implant and the injector to a minimum. This modification is especially beneficial for "endothelium in"/"tri-folded" implant technique because the lower implant interaction with the inner walls of the device allows for easier implant ejection (without the use of additional instruments like for the "pull through" technique). The surface patterning could be combined with the above mentioned coatings for the best performance.

The benefit of surface patterning is, that the contact area of the tissue graft or implant is decreased to fewer contact points when compared to a flat surface. Therefore, less tissue graft or implant surface interactions cause also less corresponding damage during tissue graft or implant loading, evaluation, storage and/or transportation as well as during ejection from the device. Importantly, the surface pattern should not interfere with the microscopy procedures during evaluation process (e.g. special alignments and dimensions of the pattern should not extensively scatter light during microscopy). Due to these circumstances, microstructured patterning as well as its combination with nanostructured patterning is considered to be the most beneficial of all named patterning types.

Therefore, in one embodiment of the present invention, the inner surface of the device comprises a surface patterning to increase the hydrophobicity and/or decrease the contact area between the tissue graft or implant, being a micro- and/or nanostructured surface pattern (combination e.g. nano-microstructuring) in the range of 100 nm to 20000 nm (0.1 µm to 20 µm), preferably between 300 nm to 5000 nm (0.3 µm to 5 µm) and more preferably between 500 nm to 2500 nm (0.5 µm to 2.5 µm).

In one embodiment of the present invention, the inner surface of the device comprises a surface patterning in combination with a hydrophobic or hydrophilic coating as described above.

The described use of the "glass-like plastic" with corresponding coatings or surface patterns for implant preparation, storage, transport and injection is more safe than glass as it does not break; as well as it is more efficient because it is designable to be implant specific. Together with the engineered shape of the device described above, it provides significant advantages in efficiency and corresponding safety of the implantation procedure of tissue grafts and implants, especially for all main corneal endothelial keratoplasty (EK) tissue graft and implant types.

In one embodiment, the device of the present invention further comprises a cap for the first opening and/or a cap for the second opening. In a most preferred embodiment, the present invention comprises only one cap.

Aiming for storage and/or transport and evaluation of the tissue, the device can be closed via applying a cap at the first opening and a cap at the second opening or via applying only one cap at the first opening. According to the invention, the cap/caps must hold tight enough to prevent the tissue graft or transplant slipping out of the device. At the same time, the cap/caps are configured such that at least the device main body, preferably the device main body and the taper area are kept uncovered for microscopy purposes.

Therefore, the present invention provides a device with a cap at the first opening and a cap at the second opening or only one cap at the first opening, wherein the caps are configured such that at least the main body of the device, preferably the main body of the device and the taper area of the device are kept uncovered.

Removal of the caps should be possible in a smooth and gentle way. The design of the caps depends on the design of the opening of the device which should be closed. Since a manifold of combinations of size and geometry of the first opening and the second opening exist according to the invention, the design of the cap must be adopted to the device design of choice.

Therefore, the present invention provides a device, wherein the design of the cap for the first opening is adopted to the design of the first opening of the device and/or the cap for the second opening of the device is adopted to the design of the second opening of the device.

The cap for closing the first opening and/or for the second opening of the device can be made of any hard or soft plastic [e.g. selected from Polypropylene (PP), Polystyrene (PS), Polyamide (PA), Polyether ketone (PEK), Polyether ether ketone (PEEK), Poly(methyl methacrylate) (PMMA), Polyethylene (PE), Polyethylene terephthalate (PET), Polybutylene terephthalate (PBT), Polyphthalamide (PPA), Polyphenylene Sulfide (PPS), Polyamide-imides (PAI), Polyphenylsulfone (PPSU), Polyethersulfone (PES), Polysulfone (PSU), Polycarbonate (PC), Acrylonitrile butadiene styrene copolymer (ABS), Styrene-acrylonitrile copolymer (SAN) or Polyvinyl chloride (PVC)], rubber, silicone, macroporous material, glass and suitable mixtures or combinations thereof.

During transport and evaluation the device is stored in a liquid transport medium. To assure sufficient supply of the tissue graft or implant (being located inside the device) with oxygen and nutrients, exchange of solvent between the inner hollow compartment of the device and the solution surrounding the device must be ensured. Therefore, in one embodiment of the invention, at least one cap can be permeable.

The permeability can be ensured via the use of small holes within the closing caps, the use of nets, membranes or macroporous materials or related permeable materials within the caps. In one embodiment of the invention, the cap closing the first opening of the device is permeable. In one embodiment of the invention, the cap closing the second opening of the device is permeable. In another embodiment, the caps closing the first and the second opening of the device are permeable.

In a most preferred embodiment of the invention, only one cap at the first opening is closing the device. In this event, exchange of solvent between the inner hollow compartment of the device and the solution surrounding the device is enabled via the second opening, thereby assuring sufficient supply of the tissue graft or implant (being located inside the device) with oxygen and nutrients. Consequently, if only the first opening is closed by a cap, this cap can, but must not, be permeable. Therefore, in a preferred embodiment of the invention, the device comprises one cap for the first opening of the device.

### Assembly for tissue graft or implant loading, storage and transport

The device alone is not suitable for successful tissue graft or implant loading into the device, there is a need for additional tools and accessories. Therefore in a second aspect, the invention relates to an assembly for loading of the tissue graft or implant, which comprises the device according to the invention, at least one syringe, a tube and at least one cap to close at least one opening of the device. The tissue graft or implant loading assembly is preferably provided in accordance with the regulations for the use in clinical practice.

For the process of tissue loading, the device is preferably connected to a tissue loading accessory. The tissue loading accessory can be for example a laboratory dish/petri dish containing the tissue graft or implant in a suitable liquid medium (e.g. BSS). Therefore, the second opening of the device is attached to a standard syringe for tissue loading via an additional tool. A standard syringe means a syringe which can for example be connected to a Luer Slip or a Luer Lock connection. According to the invention, the additional tool can be a tube.

Therefore, in one embodiment of the present invention, an assembly for loading, short-term (<24 h) storage and/or transport of a tissue graft or implant is provided, comprising
- the device according to the first aspect of the invention,
- at least one syringe,
- a tube, and
- at least one cap.

The tube is preferably made of a flexible material. In one embodiment of the invention, the tube is preferably made of a material selected from rubber, silicone, latex or related flexible materials.

The inner diameter of the tube is adjusted to be slightly smaller, e.g. up to 10 % or 20 % smaller, than the outer size of the second opening to ensure tight sealing upon attachment. The tube has preferably an inner diameter between 1 mm and 4 mm, more preferably between 2 mm and 3 mm or 10 % smaller. In a most preferred embodiment of the invention, the inner diameter of the tube is 2 mm or 10 % smaller.

The length of the tube is adjusted to be long enough for convenient handling, but short enough to prevent excessive bending of the tube which affects smooth handling. The length of the tube is preferably between 50 mm and 150 mm, more preferably between 70 mm and 130 mm, even more preferably between 80 mm and 105 mm. In a most preferred embodiment of the invention, the tube has a length of 95 mm. However, the tube can be manually shortened (e.g. with a scissor, scalpel or comparable) to the desired length for the user's convenience.

The tube according to the invention can be either directly attached to a standard syringe, e.g. by a Luer Lock or Luer Slip, or through a standard tube connector. A standard tube connector is for example a Luer connector. Such a standard connector can be made out of any soft or hard plastic, for example selected from Polypropylene (PP), Polystyrene (PS), Polyamide (PA), Polyether ketone (PEK), Polyether ether ketone (PEEK), Poly(methyl methacrylate) (PMMA), Polyethylene (PE), Polyethylene terephthalate (PET), Polybutylene terephthalate (PBT), Polyphthalamide (PPA), Polyphenylene Sulfide (PPS), Polyamide-imides (PAI), Polyphenylsulfone (PPSU), Polyethersulfone (PES), Polysulfone (PSU), Polycarbonate (PC), Acrylonitrile butadiene styrene copolymer (ABS), Styrene-acrylonitrile copolymer (SAN) or Polyvinyl chloride (PVC).

Therefore, in one embodiment of the invention, an assembly for loading, short-term (<24 h) storage and/or transport of a tissue graft or implant comprises
- the device according to the first aspect of the invention,
- at least one syringe,
- a tube, wherein the tube has a Luer Lock or a Luer Slip connector at one side and
- at least one cap
is provided.

The device is designed in a way that the loading of thin tissue grafts or implants such as DMEK/PDEK, ultrathin-/nanothin-DSEK/DSAEK grafts, and any related grafts or implants can be performed from the first opening. This opening is not only suitable for direct syringe nozzle and/or tube attachment, but also dimensioned suitable for gentle tissue graft or implant uptake due to its funnel-like design (the diameter of the opening increases slightly towards the edge).

For loading the tissue graft or implant into the device, a syringe which is for example preferably filled with BSS or liquid nutrition medium, is connected to the second opening of the device by attaching one end of the tube to the second opening and the other end of the tube (comprising the Luer Lock or Luer Slip connector) to the syringe. Subsequently, the syringe plunger is moved to push out excess air from the device and the tube. There should be no air bubbles left in the device and in the tube. For the example of DMEK tissue loading, the first opening of the device is gently placed on top of the tissue graft or implant (which is presented inside a laboratory dish/petri dish containing the tissue graft or implant in a suitable liquid medium [e.g. BSS]), without touching the tissue graft or implant, and the syringe plunger is moved to upload the tissue graft or implant inside the device by use of hydrodynamic flow.

For direct introduction of the tissue graft or implant into a living body, the tissue graft or implant can be partially positioned inside the taper area of the device, which can be called 'injection position'. In this position, the tissue graft or implant is partially rolled in or slightly compressed, which ensures stable positioning inside the device. Aiming for direct introduction of the tissue graft into a living body, another liquid-filled (e.g. BSS) syringe can be directly attached to the first opening of the device. Alternatively, the tissue graft or implant could be positioned also completely inside the taper area for the 'injection position'. However, this increases the probability of unwanted slipping out of the tissue graft or implant from the device during attachment of a syringe at the first opening of the device since in this case there is not much space left towards the second opening of the device. Thus, preferably the tissue graft or implant is partially positioned inside the taper area of the device for the 'injection position'.

Now, the tube at the second opening together with the syringe can be gently removed and the device is ready for tissue graft or implant ejection. Alternatively, this assembly comprising the device with the tissue graft or implant, two syringes each attached to the first and the second opening of the device (for the second opening, via a tube and a syringe connector), can be used for gentle in-house transport and/or storage, for example from the tissue preparation facility into the OR, if those facilities are in the same building.

For evaluation, storage and/or transport, the tissue graft or implant can be positioned inside the main body of the device, which can be called 'transport position'. For secure transport, the tubing at the second opening of the device is gently removed and at least one opening of the device is sealed by a cap, preferably the first opening is sealed by a suitable cap. Alternatively, a suitable cap seals both the first opening and the second opening. Properties of suitable caps are described above.

The device loaded with the tissue graft or implant has to be delivered in a liquid medium with a volume of at least 20 milliliters, which by far exceeds the inner volume of the device itself. Therefore, in one embodiment of the invention, the assembly for loading, storage and/or transport of a tissue graft or implant further comprises a transport device. The transport device is preferably provided in accordance with the regulations for the use in clinical practice.

Currently, only a few transport devices are available in the art. The Straiko Modified Jones Tube [24, 25] is proposed to be delivered in a viewing chamber similar to the one used for whole cornea tissue delivery, and the Geuder injector [27] is proposed to be delivered in a standard container, wherein a standard container means a standard tissue culture flask. Both transport devices are also suitable for transportation of the device of the present invention. Therefore, in one embodiment of the invention, the storage/transport device comprises a viewing chamber as described in [24, 25] or a standard tissue culture flask as described in [27].

In a preferred embodiment of the invention, the storage/transport device is a device holder, which is suitable to be positioned inside a container. Such a container can be a standard tissue culture flask, a tissue culture flask with two openings or a comparable container, such as a viewing chamber as described in [24, 25]. Standard tissue culture flask are commonly used for cell and tissue applications in research and clinics and also suitable for tissue evaluation via precision microscopy. Alternatively, this container can be a tissue culture flask with two openings, where one opening is suited for insertion of the device holder including the device and the second opening is suited for direct aspiration of the liquid medium (e.g. for fungal tests) from the inside of the device.

According to the invention, the device holder comprises
- a round neck with a holder,
- finger tips which are attached on an oval shaped platform with a cavity, and
- a tilted stripe.

The device holder according to the invention is suitable to be positioned inside a container, such as a standard tissue culture flask or a tissue culture flask with two openings.

The finger tips according to the invention tightly hold the device. Therefore, the finger tips have cavities with a shape matching the size of the device for easy assembly and gentle device removal. Advantageously, the finger tips are made from plastic which is slightly elastic (e.g. selected from Polypropylene (PP), Polyamide (PA), Polyether ketone (PEK), Polyether ether ketone (PEEK), Poly(methyl methacrylate) (PMMA), Polyethylene (PE), Polyethylene terephthalate (PET) or plastic blends, plastic mixtures, copolymers or other combinations of the mentioned plastics, preferably from PP, PA, PE or PMMA). This allows the finger tips to be used as "clips" during insertion of the device. Furthermore, the shape of the fingertips secures the orientation/position of the device during transportation. Especially, this prevents the device from spinning. The finger tips are attached on an oval shaped holder comprising a cavity, preferably made from the same material as the finger tips which is a strong support to the finger tips. The cavity is designed in a way that it does not cover the main body, preferably the main body and the taper area of the device, which are used as microscope examination area. Therefore, if the device holder is inserted into a container, such as a standard tissue culture flask or a tissue culture flask with two openings, the tissue graft or implant loaded into the device can be easily examined from both the top and bottom part of a standard tissue culture flask or of a tissue culture flask with two openings.

The oval holder with the fingers tips is attached to a tilted stripe which in its shape is matching the bottom of the standard tissue culture flask or the tissue culture flask with two openings. The tilted stripe is advantageously made from plastic, preferably made from the same material as the finger tips and the oval shaped holder with a cavity.

The tilted stripe is attached to a round neck, which from one side allows the insertion into a standard tissue culture flask or into a tissue culture flask with two openings, but from another side holds tight to the neck of the neck part of the standard tissue culture flask or to one neck part of the tissue culture flask with two openings. This prevents the movements of the whole transport device inside of the standard tissue culture flask or inside the tissue culture flask with two openings. The round neck has a holder in the center, which allows its manipulation with forceps and/or the end user's fingers, for secure and easy insertion and removal of the device holder from the standard tissue culture flask or from the tissue culture flask with two openings. The overall dimensions of the device holder are designed in a way that the one end of the tilted stripe touches the bottom of the standard tissue culture flask or the bottom of the tissue culture flask with two openings, while the other end with the round neck is in the neck of the standard tissue culture flask or in one neck of the tissue culture flask with two openings and touching the closing cap of the standard tissue culture flask or one closing cap of the tissue culture flask with two openings when the standard tissue culture flask or the tissue culture flask with two openings is closed.

In a preferred embodiment of the invention the device holder is made from plastic, preferably selected from Polypropylene (PP), Polyamide (PA), Polyether ketone (PEK), Polyether ether ketone (PEEK), Poly(methyl methacrylate) (PMMA), Polyethylene (PE), Polyethylene terephthalate (PET) or plastic blends, plastic mixtures, copolymers or other combinations of the mentioned plastics and more preferably from PP, PA, PE or PMMA. In a most preferred embodiment, the device holder is made from plastic as one-piece holder, for example via injection molding or 3D printing.

In one embodiment of the invention the round neck, the holder, the finger tips attached on the oval shaped platform with a cavity and the tilted stripe can be made from different plastic, preferably the plastic is selected from Polypropylene (PP), Polyamide (PA), Polyether ketone (PEK), Polyether ether ketone (PEEK), Poly(methyl methacrylate) (PMMA), Polyethylene (PE), Polyethylene terephthalate (PET) or plastic blends, plastic mixtures, copolymers or other combinations of the mentioned plastics and more preferably from PP, PA, PE or PMMA.

The features of the device holder according to the invention prevent any movements of the device holder inside a standard tissue culture flask or inside a tissue culture flask with two openings. The shape of the round neck prevents the horizontal movements of the device holder and the shape of the tilted stripe prevents any spinning or turning of the device holder inside of the standard tissue culture flask or inside the tissue culture flask with two openings. Since the device according to the invention is tightly hold by the finger tips of the device holder, the device holder secures absolute positioning of the device inside of the standard tissue culture flask or inside the tissue culture flask with two openings. Hence, spinning or horizontal movement of the device as well as of the tissue graft or implant inside the device is prevented. The device holder according to the invention guarantees that both the device as well as the tissue graft or implant inside the device hold the position during storage and/or transportation. For example, this is beneficial during transportation prior an operation, as graft preparation is performed by technical controlling staff in eye/tissue banks.

Advantageously, at the same time the device holder according to the invention allows visual examination control of the tissue graft or implant inside the device and inside the standard tissue culture flask or inside the tissue culture flask with two openings. It also allows an effective examination of the tissue graft or implant by different microscopy methods (tissue quality control) like specular microscopy, slit-lamp examination, light microscopy and optical coherence tomography. This is based on the transparency of the standard tissue culture flask (or of the tissue culture flask with two openings) and its common use in microscopy as well as the secured position of the device comprising the tissue graft or implant, facing the top and the bottom of the standard tissue culture flask or the bottom of the tissue culture flask with two openings with the flat surfaces of its main body and taper area. This invention is also very cost effective as it allows the use of commercially available standard tissue culture flasks while the device holder could be made from plastic as one part, e.g. via injection molding or 3D printing. Furthermore, the device holder has the advantage that at the same time very secured inclusion and removing of the device is enabled which can even be done by untrained staff.

### Washing Assembly

Further, a washing assembly for washing and staining a tissue graft or implant inside of the device prior ejection (e.g. implantation into an eye) is provided. According to the invention, the washing assembly comprises a macroporous material, the device according to the invention, at least one syringe, optionally at least one cap, and a 2-way extension line or a 3-way stopcock where both can comprise flexible tubes. The washing assembly is preferably provided in accordance with the regulations for the use in clinical practice.

Since the tissue graft or implant is delivered in liquid nutrition medium as described above, it must at least be washed with BSS and/or stained with trypan blue and washed with BSS prior the injection to the living body. Trypan blue is a dye which is used for better visualization of the tissue graft or implant, especially for ophthalmological interventions. Therefore, it is suitable for quality control of tissue grafts and implants. Due to cell toxicity issues, the tissue graft or implant should not be exposed to trypan blue and BSS for a long time so the tissue graft or implant is usually stained and washed right prior the implantation. The staining and washing is a complex procedure as it should be done without damaging the tissue graft or implant. The staining and washing is most preferably performed in the device where the tissue graft or implant is delivered with, in order to decrease the tissue graft or implant manipulation, which could result to tissue graft or implant disorientation, loss or damage. Currently, this requires an advanced training of the surgeon. Nevertheless, sometimes an unwanted release of the tissue graft or implant from the delivery devices requires its time-consuming and cell-damaging reinsertion back into the device [13].

Therefore, the present invention furthermore provides an assembly for gentle and secured tissue graft or implant washing. The washing assembly comprises
- a macroporous material,
- the device according to the first aspect of the invention or comparable conventional devices,
- at least one syringe,
- optionally at least one cap,
- optionally at least one tube clamp,
- a 2-way extension line or a 3-way stopcock, wherein both optionally comprise flexible tubes.

In one embodiment of the invention, the washing assembly comprises a macroporous material to control the liquid flow during washing of the tissue graft or implant. Due to interconnected pores, the macroporous materials allows a liquid flow if a force is applied, but generally prevents the liquid flow in a steady state. In other words, when the second opening of the device faces a macroporous material, a liquid which enters the device from the first opening passes the device and can pass through the macroporous material, but any large objects such as a tissue graft or implant cannot escape. This assembly allows fast and secure staining and washing of the tissue graft or implant even by unexperienced staff because of its simple handling. Importantly, the liquid cannot escape from the macroporous material unless external force is applied so the liquid medium and following staining solution will not return back to the device keeping the tissue graft or implant clean ("sponge effect"). This is analogous to a sponge in which water from the sponge could be removed only when the sponge is compressed. Importantly, using macroporous materials for washing does not impair the viability of the transferred tissue graft or implant, e.g. of human corneal endothelial cells.

In one embodiment of the invention, the macroporous material is embedded within a stable container. The macroporous materials may either be not fixed or fixed to the stable container, e.g. by the application of surface functionalization, special surface topography, glues, stiches, or special haptic/geometry of the container.

In another embodiment of the invention, the macroporous material is provided without any kind of container.

The macroporous material can have any possible geometry, preferably the macroporous material has at least one flat surface. The minimum thickness of the macroporous material depends on its total volume. Preferably, the shape of the macroporous material is a cylinder with a diameter between 1 cm to 5 cm and a height between 0.3 cm to 3 cm (volume of circa 0.25 milliliters to 58 milliliters.) This has the advantage that the second opening of the device can be pressed against the flat surface of the macroporous material ensuring a tight connection.

Since the second opening of the device can have different shapes and geometries as described above (e.g. elliptical, round, biconvex or rounded rectangle shape), in an even more preferred embodiment of the invention, one surface of the macroporous material is adapted to the shape and geometry of the second opening of the device. This embodiment has the advantage that a very tight connection between the macroporous material and the second opening of the device is achieved easily.

The macroporous material suitable to be used in the washing assembly of the invention has interconnected pores and a porosity between 10 µm and 600 µm, preferably between 10 µm and 400 µm, more preferably between 30 µm and 300 µm. A macroporous material has an advantageously low bulk stiffness and a high stability upon compression, which makes the handling during a washing and staining process according to the invention easier. Furthermore, it allows an easy lifting up of the device due to the "sponge effect" of the macroporous material described above.

According to the invention, the macroporous material can be made of any type of suitable material, including natural sponges, foams, pure synthetic or biological polymers, synthetic or biological polymer blends, rubber, any combinations thereof and comparable sponge-like materials. In a preferred embodiment of the invention, the macroporous material is made from synthetic or biological polymers such as poly(vinyl alcohol)-based or cellulose-based sponges. These materials have the advantage that they are easily available since they are widely used in medical devices (even implantable) and are readily available certified for medical use with a very high level of sterility.

Moreover, the macroporous materials can be prepared according to any developed techniques [33-35], including porogen leaching [36, 37], gas foaming [38, 39], phase separation [40, 41], electrospinning [42] as well as cryogelation in aqueous media [43-45].

Macroporous materials according to the invention have a low bulk stiffness and high mechanical stability upon compression. These properties allow for an easy handling of the materials and allow for gentle geometrical adaptation to the shape of the second opening of the device. The macroporous material has a "cushioning effect", which enables gentle washing and staining of tissue grafts and implants inside the device without exerting any mechanical force that could impair the integrity of the tissue graft or implant during the staining and washing procedure.

Additionally, the washing assembly comprises either a 2-way extension line or 3-way stopcock. Both can consist of hard or soft plastic [e.g. selected from Polypropylene (PP), Polystyrene (PS), Polyamide (PA), Polyether ketone (PEK), Polyether ether ketone (PEEK), Poly(methyl methacrylate) (PMMA), Polyethylene (PE), Polyethylene terephthalate (PET), Polybutylene terephthalate (PBT), Polyphthalamide (PPA), Polyphenylene Sulfide (PPS), Polyamide-imides (PAI), Polyphenylsulfone (PPSU), Polyethersulfone (PES), Polysulfone (PSU), Polycarbonate (PC), Acrylonitrile butadiene styrene copolymer (ABS), Styrene-acrylonitrile copolymer (SAN) or Polyvinyl chloride (PVC)], rubber, silicone, glass and suitable combinations thereof and both can comprise flexible tubes. The tubes can consist of a material selected from rubber, silicone, latex or related flexible materials.

The length of the tubes is adjusted to be long enough for convenient handling, but short enough to prevent excessive bending of the tube which affects smooth handling. The length of the tubes is preferably between 100 mm and 200 mm, more preferably between 120 mm and 180 mm, even more preferably between 140 mm and 160 mm. In a very preferred embodiment of the invention, the tubes have a length of 150 mm.

In one embodiment of the invention, the washing assembly further comprises tube clamps and closing caps for the device and the endings of the 2-way extension line or 3-way stopcock. The tube clamps can be used to interrupt the flow inside a tube used in the washing assembly. The caps are suitable to close open endings of the 2-way extension line or the 3-way stopcock. A closing cap for the device could be used to close the second opening of the device during and/or after washing and staining and/or to close the first opening of the device after disconnecting the 2-way extension line or the 3-way stopcock from the device. In the case of a permeable closing cap, the cap could remain on the second opening of the device even during washing and staining procedure.

According to the invention, the 2-way extension line as well as the 3-way stopcock are preferably equipped with connections for attaching the device and connections for attaching a syringe. In one embodiment of the invention, the 2-way extension line as well as the 3-way stopcock are preferably equipped with a flexible tube with a male connection for attaching the device via the first opening. The male connection is preferably a Luer Lock or a Luer Slip connection. Both the 2-way extension line and the 3-way stopcock have two other connections, which are preferably female connectors. The female connectors are preferably Luer Lock or Luer Slip connectors. The female connectors are suitable to connect a conventional (for example a Luer Lock or a Luer Slip) syringe. The connections with the female connectors can, but must not, be equipped with tubes and/or caps as well.

The washing assembly according to the invention further comprises a syringe, which can be connected to the 2-way extension line or the 3-way stopcock. In one embodiment of the invention, the washing assembly according to the invention comprises two syringes, wherein one of the syringes (e.g. filled with BSS) can be connected to one of the female connectors of the 2-way extension line or the 3-way stopcock and the other syringe (e.g. filled with trypan blue staining solution) can be connected to the other female connector of the 2-way extension line or the 3-way stopcock.

According to the invention, the washing assembly can comprise any comparable conventional device instead of the device according to the invention. In principal, a comparable conventional device is a device which is suitable to be used to host a tissue graft or implant. Such conventional devices are for example Straiko Modified Jones Tube [24, 25], intraocular lens cartridge [26] or Geuder DMEK injector [27].

### Method for preparing a tissue graft or implant

In a fourth aspect, the invention provides a method for preparing a tissue graft or implant ready for injection into the living body using the device according to the invention.

The method of washing the tissue graft or implant with the help of the washing assembly is described in context with a method for preparing a tissue graft or implant. Accordingly, the present invention provides a method for preparing a tissue graft or implant using the device according to claims 1 to 7, comprising the steps:
a) providing a tissue graft or implant,
b) loading the tissue graft or implant into the device,
c) sealing the device by at least one cap,
d) evaluation and quality control of the tissue graft or implant inside the device, preferably placed inside a storage/transport container by help of the device holder (e.g. for long-distance transportation outside the tissue bank) according to the invention,
e) transport of the device with the tissue graft or implant, preferably using the device holder inside a storage/transport container (e.g. for long-distance transportation outside the tissue bank) according to the invention,
f) otionally evaluation and quality control of the tissue graft or implant inside the device,
g) washing and staining of the tissue graft or implant inside the device.

According to the method of the present invention, a tissue implant or graft is provided, which is preferably a tissue graft or implant suitable for DMEK, PDEK, ultrathin-/nanothin-DSEK or DSAEK techniques or any related techniques.

The tissue graft or implant is loaded into the device according to the invention. Therefore, the device is attached to the tissue loading accessory. The tissue loading accessory can be for example a laboratory dish/petri dish containing the tissue graft or implant in a suitable liquid medium (e.g. BSS). Further steps of tissue graft or implant loading are described in the "Assembly for tissue graft or implant loading, storage and transport" section of this invention.

For evaluation, storage and/or transport and evaluation, the tissue graft or implant can be positioned inside the main body of the device, which can be called 'transport position'. For secure transport, at least one opening of the device is sealed by a cap, preferably the first opening and the second opening are sealed by suitable caps. Properties of suitable caps are described above.

According to the method of the invention, the device loaded with the tissue graft or implant is transported to a desired place. According to one embodiment of the invention, this is practiced by using a transport device according to the invention. Most preferably, a transport device comprising a device holder according to the invention is used. Therefore, the device is fixed in the device holder and the device holder is positioned in a container, such as a standard tissue culture flask or a tissue culture flask with two openings, which is dimensioned to be filled with at least 20 milliliters of a suitable liquid medium, leaving maximum 1-2 mL air inside the transport assembly, or preferably less.

Accordingly, in one embodiment of the invention, the device is transported within the device holder within a container, such as a standard tissue culture flask or a tissue culture flask with two openings. According to the method of the invention, evaluation and quality control of the tissue graft or implant is performed inside the device. Therefore, the device can stay inside the transport device and even inside the container, such as a standard tissue culture flask or a tissue culture flask with two openings if a transport device with a device holder according to the invention is used. According to the invention, the main body and taper area of the device are suitable for performing the above mentioned precision microscopy techniques such as specular microscopy, slit-lamp examination, light microscopy and optical coherence tomography.

It is advantageously that evaluation and quality control can be performed with the device containing the tissue graft or implant being inside the transport device and inside the container, such as a standard tissue culture flask or a tissue culture flask with two openings because additional steps of manipulation of the tissue graft or implant are avoided. Thus, a possible damage of the tissue graft or implant is minimized.

Before use/implantation, the tissue graft or implant must be washed and preferably stained. According to the invention, this is preferably performed using the washing assembly provided by the invention and described above. In other words, in one embodiment of the invention, step g) is performed while the tissue graft or implant is located inside the device. Therefore, the device is removed from the transport device and closing caps are removed at least from the first opening of the device and optionally also from the second opening of the device, if the cap at the second opening of the device is not permeable for liquids.

Prior tissue staining and washing, one syringe filled with BSS is attached to one female connector of the 2-way extension line or the 3-way stopcock and one syringe filled with staining solution (e.g. trypan blue) is attached to the other female connector. The volume of the BSS-filled syringe is preferably greater than the volume for the second staining solution-filled syringe. In one embodiment of the invention, the volume of the BSS-filled syringe is between 5 ml and 10 ml and the volume of the staining solution-filled syringe is between 1 ml and 4 ml.

For removing air from the assembly, in the case of using a 2-way extension line equipped with tubing at both female connectors, the clamp at the position with the staining solution-filled syringe remains open, while another clamp at the BSS-filled syringe position outlet is fully closing the tube. For the 3-way stopcock, the tap position is adjusted in a way that the staining solution-filled syringe and the male connection outlet are connected while the position with the BSS-filled syringe remains closed.

First, the tubing attached to the staining solution-filled syringe is fully flushed with staining solution. The staining solution must not enter the male connection of the assembly since this step is only needed for air removal.

Next, complete air removal and flush of the assembly with BSS solution needs to be performed. In the case of using a 2-way extension line equipped with tubing at both female connectors, the clamp at the position with the staining solution-filled syringe is fully closing the tube, while another clamp at the BSS-filled syringe position outlet remains open. For the 3-way stopcock, the tap position is adjusted in a way that the BSS-filled syringe and the male connection outlet are connected while the position with the staining solution-filled syringe remains closed.

Now, the assembly is fully flushed with BSS by pushing the plunger of the BSS-filled syringe. There should be no air left in the assembly as well as there should be no staining solution eluting from the male connection of the assembly. Fourth, the device containing the tissue graft or implant is attached via the first opening to the male connector of the 2-way extension line or 3-way stopcock and placed into a BSS-filled laboratory dish/petri dish to prevent air infiltration to the device.

To decrease the probability of tissue graft or implant slipping out of the device, the second opening of the device can be closed by a liquid-permeable cap or can be placed onto/into the macroporous material from the washing assembly during the whole staining and washing procedure. If applicable, liquid transport medium can now be flushed out of the device prior staining by gently pushing the plunger of the BSS-filled syringe. Alternatively, staining of the tissue graft or implant can be performed without prior washing with BSS solution.

For this, in the case of using a 2-way extension line equipped with tubing at both female connectors, the clamp at the BSS-filled syringe position is fully closing the tube, while the other clamp at the position with the staining solution-filled syringe outlet remains open. In case of the use of a 3-way stopcock, the tap position is adjusted in a way that the staining solution-filled syringe and the male connection outlet are connected, while the position with the BSS-filled syringe remains closed.

Now, the staining solution is gently added to the tissue graft or implant in the device by pushing the plunger of the staining solution-filled syringe and left for at least 1-2 minutes for sufficient staining. Extended staining will cause increased cell damage. After staining, in the case of using a 2-way extension line equipped with tubing at both female connectors, the clamp at the position with the staining solution-filled syringe is fully closing the tube, while the other clamp at the BSS-filled syringe position outlet remains open. For the 3-way stopcock, the tap position is adjusted in a way that the BSS-filled syringe and the male connection outlet are connected, while the position with the staining solution-filled syringe remains closed.

Lastly, the device is fully flushed with BSS by pushing the plunger of the BSS-filled syringe. The solution in the device should be transparent and no staining solution should remain.

Now, the device and the respective (washed and/or stained and washed) tissue graft or implant is ready for implantation into the living body. For this, the device can remain at the washing assembly prior insertion into the living body to save time and decrease probability of tissue graft or implant slipping out of the device. Alternatively, the device can be gently removed from the washing assembly and the first opening of the device can be attached to a BSS-filled conventional (for example a Luer Lock or a Luer Slip) syringe prior insertion into the living body, which is described above for the tissue graft or implant loading procedure. In the case of using a cap attached to the second opening of the device during staining and washing procedure, the cap can remain attached to the device to decrease the probability of tissue graft or implant slipping out of the device, but must be removed right prior implantation into the living body.

Upon applying a hydrodynamic pressure, e.g. via the syringe, the tissue graft or implant can gently be washed without to be "pushed" out from the device. This can be performed without extensive manipulation of the surgeon and without application of forceps or any other surgical devices. Gentle and slow plunger movement of the syringes will prevent tissue graft or implant slipping.

However, in the case the tissue graft or implant moves within the device during the staining and washing procedure, the tissue graft or implant can be re-positioned inside the device by slight pulling the plunger of the respective syringe at the female connector position of the washing assembly. Importantly, there should be no air entering the device during graft re-positioning inside the device.

The tissue graft or implant can be released out of the device by pushing the plunger of the BSS-filled syringe. In the same way, the tissue graft or implant can be injected into the living body. For insertion of the tissue graft or implant, e.g. for ophthalmological interventions, the longer wing of the chamfered part of the second opening of the device preferably faces the bottom (positioned towards the posterior eye chamber), ensuring the implant injection into the anterior chamber of the eye.

Due to the inner shape and dimensions of the device according to the invention, release or implantation of tissue grafts or implants is performed in an oriented manner. The preferably rounded rectangular inner shape of the device prevents the tissue graft or implant from turning inside the device even during the forward or backward movement along the inner hollow compartment of the device (e.g. during graft re-positioning for implantation after the transport as described above), thus assuring the stability of the tissue graft or implant orientation at all times - from tissue loading into the device until tissue release from the device.

According to the description, the present invention pertains to the following items:
1. Device for secure support storage and/or transport of a tissue graft or implant, comprising:
   - a first opening,
   - a main body,
   - a taper area, and
   - a second opening,
   wherein the main body is transparent and has a rectangle shape and the taper area is transparent and has a an elliptical, round, biconvex or rectangle shape, and wherein the main body comprises at least two flat and parallel opposite sites.
2. Device according to item 1, wherein the first opening has a round shape and a funnel-like design configured to connect with a tube or a syringe.
3. Device according to any one of items 1 to 2, wherein the second opening has an elliptical, round, biconvex or rounded rectangle shape.
4. Device according to anyone of items 1 to 3, wherein the outer wall of the second opening has a different shape than the inner wall.
5. Device according to any one of items 1 to 4, wherein the outer wall of the taper area has a different shape than the inner wall.
6. Device according to any one of items 1 to 5, comprising a diameter of the inner part of the first opening between 3 mm and 6 mm, preferably between 4 mm and 5 mm.
7. Device according to any one of items 1 to 6, comprising a distance between top wall and bottom wall, which are flat and parallel to each other of the inner part of the main body between 1 mm and 5 mm, preferably between 1.5 mm and 4 mm, more preferably between 2 mm and 3 mm.
8. Device according to any one of items 1 to 7, comprising a distance between top wall and bottom wall of the inner part of the taper area and the second opening between 0.8 mm and 2 mm, preferably between 1 mm and 2 mm.
9. Device according to any one of items 1 to 8, wherein the device has a total length in the range from 25 mm to 50 mm, preferably from 30 mm to 40 mm.
10. Device according to any one of items 1 to 9, wherein the device consists of glass, preferably borate glass.
11. Device according to any one of items 1 to 9, wherein the device consists of plastic, wherein said plastic is transparent and has a refractive index (rᵢ) in the range of rᵢ = 1.30 to rᵢ = 1.71, preferably rᵢ = 1.30 to rᵢ = 1.65, most preferably 1.30 to rᵢ = 1.60, which is similar to the rᵢ = 1.33 to 1.34 (at 20 °C) of balanced salt solution (BSS), such as Hank's BSS, Earle's BSS, Tyrode's salt solution, Alsever's salt solution, phosphate-buffered saline (PBS), Tris-buffered saline (TBS), Puck's salt solution, Gey's salt solution, Ringer's salt solution, Simm's salt solution and related buffered saline solutions.
12. Device according to item 11, wherein the device consists of polyacrylates, polycarbonates or polystyrenes.
13. Device according to any one of items 10 to 12, wherein the inner surface of the device comprises
   - a hydrophobic coating, selected from coatings comprising acrylate, organo-siloxane, silane, epoxy, a polymer, Molybdenum disulfide, Molybdenum disulfide/graphite, Tungsten disulfide or graphite, preferably selected from coatings comprising a polymer, organo-siloxane, silane, acrylate or epoxy; or
   - a hydrophilic coating, selected from coatings comprising any hydrophilic polymer/hydrogel, preferably selected from coatings comprising poly(ethylene glycol), poly(acrylate), poly(methacrylate) or a UV/photo-active polymer.
14. Device according to any one of items 10 to 12, wherein the inner surface of the device comprises a surface patterning, being a micro- or nanostructured surface pattern or a combination thereof (e.g. nano-microstructuring) in the range of 100 nm to 20000 nm, preferably between 300 nm to 5000 nm and more preferably between 500 nm to 2500 nm.
15. Device according to any one of items 1 to 14, further comprising a cap for the first opening and/or a cap for the second opening.
16. Device according to item 15, wherein the caps are configured such that at least the main body of the device, preferably the main body of the device and the taper area of the device are kept uncovered.
17. Device according to any one of items 15 to 16, wherein the design of the cap for the first opening is adopted to the design of the first opening of the device and/or the cap for the second opening of the device is adopted to the design of the second opening of the device.
18. Device according to any one of items 15 to 17, wherein the cap consists of a material selected from hard or soft plastic such as Polypropylene (PP), Polystyrene (PS), Polyamide (PA), Polyether ketone (PEK), Polyether ether ketone (PEEK), Poly(methyl methacrylate) (PMMA), Polyethylene (PE), Polyethylene terephthalate (PET), Polybutylene terephthalate (PBT), Polyphthalamide (PPA), Polyphenylene Sulfide (PPS), Polyamide-imides (PAI), Polyphenylsulfone (PPSU), Polyethersulfone (PES), Polysulfone (PSU), Polycarbonate (PC), Acrylonitrile butadiene styrene copolymer (ABS), Styrene-acrylonitrile copolymer (SAN) or Polyvinyl chloride (PVC), rubber, silicone, macroporous material, glass and possible combinations thereof.
19. Device according to any one of items 15 to 18, wherein the cap is permeable.
20. Device according to item 19, wherein the cap comprises small holes, networks, membranes or macroporous materials or other permeable materials.
21. Assembly for loading, storage and transport of a tissue graft or implant, comprising
   - the device according to items 1 to 20,
   - at least one syringe,
   - a tube, and
   - at least one cap.
22. Assembly according to item 21, wherein the tube consists of a flexible material, preferably selected from rubber, silicone, latex or related flexible materials.
23. Assembly according to any one of items 21 to 22, wherein the inner diameter of the tube is up to 10 % or 20 % smaller than the size of the second opening of the device to ensure tight sealing upon attachment and the tube has a length preferably between 50 mm and 150 mm, more preferably between 70 mm and 130 mm, even more preferably between 80 mm and 105 mm and most preferably a length of 95 mm.
24. Assembly according to any one of items 21 to 23, wherein the tube has a Luer Lock, a Luer Slip or a Luer connector at one side, wherein the connector consists of a material selected from hard or soft plastic such as Polypropylene (PP), Polystyrene (PS), Polyamide (PA), Polyether ketone (PEK), Polyether ether ketone (PEEK), Poly(methyl methacrylate) (PMMA), Polyethylene (PE), Polyethylene terephthalate (PET), Polybutylene terephthalate (PBT), Polyphthalamide (PPA), Polyphenylene Sulfide (PPS), Polyamide-imides (PAI), Polyphenylsulfone (PPSU), Polyethersulfone (PES), Polysulfone (PSU), Polycarbonate (PC), Acrylonitrile butadiene styrene copolymer (ABS), Styrene-acrylonitrile copolymer (SAN) or Polyvinyl chloride (PVC).
25. Assembly according to item 21, further comprising a transport device.
26. Assembly according to item 25, wherein the transport device is a device holder comprising
   - a round neck with a holder,
   - finger tips which are attached on an oval shaped platform with a cavity, and
   - a tilted stripe,
   which is suitable to be positioned inside a container, such as a standard tissue culture flask or a tissue culture flask with two openings.
27. Assembly according to item 26, wherein the finger tips comprise cavities with a shape which matches the side of the device.
28. Assembly according to any one of items 26 to 27, wherein the finger tips and the oval shaped platform do not optically interfere with the main body and the taper area of the device.
29. Assembly according to any one of items 26 to 28, wherein the overall dimensions of the device holder are configured that the one end of the tilted stripe is in touch with the bottom of the standard tissue culture flask or with the bottom of the tissue culture flask with two openings, while the other end with the round neck extends into the neck of the standard tissue culture flask or into one neck of a tissue culture flask with two openings and is in touch with the closing cap of the standard tissue culture flask or with one cap of the the tissue culture flask with two openings when the standard tissue culture flask or the tissue culture flask with two openings is closed.
30. Assembly according to any one of items 26 to 29, wherein the parts of the device holder consist of plastic selected from Polypropylene (PP), Polyamide (PA), Polyether ketone (PEK), Polyether ether ketone (PEEK), Poly(methyl methacrylate) (PMMA), Polyethylene (PE), Polyethylene terephthalate (PET), preferably from PP, PA, PE or PMMA.
31. Assembly according to any one of items 26 to 30, wherein the device holder is a one-piece holder, for example prepared by injection molding or 3D printing.
32. Washing assembly for washing and staining a tissue graft or implant, comprising
   - a macroporous material,
   - the device according to items 1 to 20 or comparable conventional devices,
   - at least one syringe,
   - optionally at least one cap,
   - optionally at least one tube clamp, and
   - a 2-way extension line or a 3-way stopcock, wherein both optionally comprise flexible tubes.
33. Washing assembly according to item 32, wherein the 2-way extension line or the 3-way stopcock consists of a material selected from hard or soft plastic such as Polypropylene (PP), Polystyrene (PS), Polyamide (PA), Polyether ketone (PEK), Polyether ether ketone (PEEK), Poly(methyl methacrylate) (PMMA), Polyethylene (PE), Polyethylene terephthalate (PET), Polybutylene terephthalate (PBT), Polyphthalamide (PPA), Polyphenylene Sulfide (PPS), Polyamide-imides (PAI), Polyphenylsulfone (PPSU), Polyethersulfone (PES), Polysulfone (PSU), Polycarbonate (PC), Acrylonitrile butadiene styrene copolymer (ABS), Styrene-acrylonitrile copolymer (SAN) or Polyvinyl chloride (PVC), silicone, glass and possible combinations thereof.
34. Washing assembly according to any one of items 32 to 33, wherein the macroporous material has at least one flat surface.
35. Washing assembly according to any one of items 32 to 34, wherein the macroporous material is enclosing the second opening of the device and is adapted to the shape of the second opening of the device.
36. Washing assembly according to any one of items 32 to 35, wherein the macroporous material comprises interconnected pores and a porosity between 10 µm and 600 µm, preferably between 10 µm and 400 µm, more preferably between 30 µm and 300 µm.
37. Washing assembly according to any one of items 32 to 36, wherein the macroporous material consists of a material selected from natural sponges, foams, pure synthetic or biological polymers, synthetic or biological polymer blends, rubber, combinations thereof, and comparable sponge-like materials.
38. Washing assembly according to any one of items 36 to 37, wherein the macroporous material consists of a synthetic or biological polymers such as poly(vinyl alcohol)-based or cellulose-based sponges.
39. Washing assembly according to any one of items 32 to 38, wherein the macroporous material is embedded in a stable container.
40. Method for preparing a tissue graft or implant using the device according to items 1 to 20, comprising the steps:
   a) providing a tissue graft or implant,
   b) loading the tissue graft or implant into the device,
   c) sealing the device by at least one cap,
   d) evaluation and quality control of the tissue graft or implant inside the device,
   e) transport of the device with the tissue graft or implant,
   f) optionally evaluation and quality control of the tissue graft or implant inside the device,
   g) washing and staining of the tissue graft or implant inside the device.
41. Method according to item 40, wherein the device is transported within the device holder.
42. Method according to items 40 to 41, wherein the device is transported within the device holder within a container, such as a standard tissue culture flask or a tissue culture flask with two openings.
43. Method according to any one of items 40 to 42, wherein evaluation and quality control of the tissue graft or implant is performed with the device containing the tissue graft or implant being inside the transport device and inside a container, such as a standard tissue culture flask or a tissue culture flask with two openings.
44. Method according to any one of items 40 to 43, wherein washing and staining of the tissue graft or implant is performed with the washing assembly, while the tissue graft or implant is located inside the device.

### Tables

In the following the invention is further described by 3 tables, wherein
- **Table 1:**: shows plastic compositions which are suitable for the device, where any listed plastic, plastic blends, plastic mixtures, copolymers or other combinations of the listed plastics can be used,
- **Table 2:**: shows hydrophobic coatings for the inner compartment of the device, where any listed coatings, coating blends, coating mixtures, or other combinations of the listed coatings can be used,
- **Table 3:**: shows hydrophilic coatings for the inner compartment of the device, where any listed coatings, coating blends, coating mixtures, or other combinations of the listed coatings can be used.

**Table 1**

| **Polymer** | **Refractive Index (at 620 nm and 23 °C)** |
|---|---|
| Poly(hexafluoropropylene oxide) | 1.3010 |
| Poly(tetrafluoroethylene-co-hexafluoropropylene) | 1.3380 |
| Poly(pentadecafluorooctyl acrylate) | 1.3390 |
| Poly(tetrafluoro-3-(heptafluoropropoxy)propyl acrylate) | 1.3460 |
| Poly(tetrafluoro-3-(pentafluoroethoxy)propyl acrylate) | 1.3480 |
| Poly(tetrafluoroethylene) | 1.3500 |
| Poly(undecafluorohexyl acrylate) | 1.3560 |
| Poly(nonafluoropentyl acrylate) | 1.3600 |
| Poly(tetrafluoro-3-(trifluoromethoxy)propyl acrylate) | 1.3600 |
| Poly(pentafluorovinyl propionate) | 1.3640 |
| Poly(heptafluorobutyl acrylate) | 1.3670 |
| Poly(trifluorovinyl acetate) | 1.3750 |
| Poly(octafluoropentyl acrylate) | 1.3800 |
| Poly(methyl 3,3,3-trifluoropropyl siloxane) | 1.3830 |
| Poly(pentafluoropropyl acrylate) | 1.3850 |
| Poly(2-heptafluorobutoxy)ethyl acrylate) | 1.3900 |
| Poly(chlorotrifluoroethylene) | 1.3900 |
| Poly(2,2,3,4,4-hexafluorobutyl acrylate) | 1.3920 |
| Poly(methyl hydro siloxane) | 1.3970 |
| Poly(methacrylic acid), sodium salt | 1.4010 |
| Poly(dimethyl siloxane) | 1.4035 |
| Poly(trifluoroethyl acrylate) | 1.4070 |
| Poly(2-(1,1,2,2-tetrafluoroethoxy)ethyl acrylate | 1.4120 |
| Poly(trifluoroisopropyl methacrylate) | 1.4177 |
| Poly(2,2,2-trifluoro-1-methylethyl methacrylate) | 1.4185 |
| Poly(2-trifluoroethoxyethyl acrylate) | 1.4190 |
| Poly(vinylidene fluoride) | 1.4200 |
| Poly(trifluoroethyl methacrylate) | 1.4370 |
| Poly(methyl octadecyl siloxane) | 1.4430 |
| Poly(methyl hexyl siloxane) | 1.4430 |
| Poly(methyl octyl siloxane) | 1.4450 |
| Poly(isobutyl methacrylate) | 1.4470 |
| Poly(vinyl isobutyl ether) | 1.4507 |
| Poly(methyl hexadecyl siloxane) | 1.4510 |
| Poly(ethylene oxide) | 1.4539 |
| Poly(vinyl ethyl ether) | 1.4540 |
| Poly(methyl tetradecyl siloxane) | 1.4550 |
| Poly(ethylene glycol mono-methyl ether) | 1.4555 |
| Poly(vinyl n-butyl ether) | 1.4563 |
| Poly(propylene oxide) | 1.4570 |
| Poly(3-butoxypropylene oxide) | 1.4580 |
| Poly(3-hexoxypropylene oxide) | 1.4590 |
| Poly(ethylene glycol) | 1.4590 |
| Poly(vinyl n-pentyl ether) | 1.4590 |
| Poly(vinyl n-hexyl ether) | 1.4591 |
| Poly(4-fluoro-2-trifluoromethylstyrene) | 1.4600 |
| Poly(vinyl octyl ether) | 1.4613 |
| Poly(vinyl n-octyl acrylate) | 1.4613 |
| Poly(vinyl 2-ethylhexyl ether) | 1.4626 |
| Poly(vinyl n-decyl ether) | 1.4628 |
| Poly(2-methoxyethyl acrylate) | 1.4630 |
| Poly(acryloxypropyl methyl siloxane) | 1.4630 |
| Poly(4-methyl-1-pentene) | 1.4630 |
| Poly(3-methoxypropylene oxide) | 1.4630 |
| Poly(t-butyl methacrylate) | 1.4638 |
| Poly(vinyl n-dodecyl ether) | 1.4640 |
| Poly(3-ethoxypropyl acrylate) | 1.4650 |
| Poly(vinyl propionate) | 1.4664 |
| Poly(vinyl acetate) | 1.4665 |
| Poly(vinyl propionate) | 1.4665 |
| Poly(vinyl methyl ether) | 1.4670 |
| Poly(ethyl acrylate) | 1.4685 |
| Poly(vinyl methyl ether) (isotactic) | 1.4700 |
| Poly(3-methoxypropyl acrylate) | 1.4710 |
| Poly(1-octadecene) | 1.4710 |
| Poly(2-ethoxyethyl acrylate) | 1.4710 |
| Poly(isopropyl acrylate) | 1.4728 |
| Poly(1-decene) | 1.4730 |
| Poly(propylene) (atactic) | 1.4735 |
| Poly(lauryl methacrylate) | 1.4740 |
| Poly(vinyl sec-butyl ether) (isotactic) | 1.4740 |
| Poly(n-butyl acrylate) | 1.4740 |
| Poly(dodecyl methacrylate) | 1.4740 |
| Poly(ethylene succinate) | 1.4744 |
| Poly(tetradecyl methacrylate) | 1.4746 |
| Poly(hexadecyl methacrylate) | 1.4750 |
| Cellulose acetate butyrate | 1.4750 |
| Cellulose acetate | 1.4750 |
| Poly(vinyl formate) | 1.4757 |
| Ethylene/vinyl acetate copolymer-40% vinyl acetate | 1.4760 |
| Poly(2-fluoroethyl methacrylate) | 1.4768 |
| Poly(octyl methyl silane) | 1.4780 |
| Ethyl cellulose | 1.4790 |
| Poly(methyl acrylate) | 1.4793 |
| Poly(dicyanopropyl siloxane) | 1.4800 |
| Poly(oxymethylene) | 1.4800 |
| Poly(sec-butyl methacrylate) | 1.4800 |
| Poly(dimethylsiloxane-co-alpha-methyl styrene) | 1.4800 |
| Poly(n-hexyl methacrylate) | 1.4813 |
| Ethylene/vinyl acetate copolymer-33% vinyl acetate | 1.4820 |
| Poly(n-butyl methacrylate) | 1.4830 |
| Poly(ethylidene dimethacrylate) | 1.4831 |
| Poly(2-ethoxyethyl methacrylate) | 1.4833 |
| Poly(n-propyl methacrylate) | 1.4840 |
| Poly(ethylene maleate) | 1.4840 |
| Ethylene/vinyl acetate copolymer-28% vinyl acetate | 1.4845 |
| Poly(ethyl methacrylate) | 1.4850 |
| Poly(vinyl butyral) | 1.4850 |
| Poly(vinyl butyral)-11% hydroxyl | 1.4850 |
| Poly(3,3,5-trimethylcyclohexyl methacrylate) | 1.4850 |
| Poly(2-nitro-2-methylpropyl methacrylate) | 1.4868 |
| Poly(dimethylsiloxane-co-diphenylsiloxane) | 1.4880 |
| Poly(1,1-diethylpropyl methacrylate) | 1.4889 |
| Poly(triethylcarbinyl methacrylate) | 1.4889 |
| Poly(methyl methacrylate) | 1.4893 |
| Acrylite® | 1.4893 |
| Lucite® | 1.4893 |
| R-Cast® | 1.4893 |
| Plexiglas® | 1.4893 |
| Perspex® | 1.4893 |
| Perspex® | 1.4893 |
| Oroglas® | 1.4893 |
| Altuglas® | 1.4893 |
| Cyrolite® | 1.4893 |
| Sumipex® | 1.4893 |
| Vitroflex® | 1.4893 |
| LIMACRYL | 1.4893 |
| Per-Clax® | 1.4893 |
| Polycast™ | 1.4893 |
| PLAZCRYL | 1.4893 |
| Poly(2-decyl-1,4-butadiene) | 1.4899 |
| Polypropylene, isotactic | 1.4900 |
| Poly(vinyl butyral)-19% hydroxyl | 1.4900 |
| Poly(mercaptopropyl methyl siloxane) | 1.4900 |
| Poly(ethyl glycolate methacrylate) | 1.4903 |
| Poly(3-methylcyclohexyl methacrylate) | 1.4947 |
| Poly(cyclohexyl alpha-ethoxyacrylate) | 1.4969 |
| Methyl cellulose | 1.4970 |
| Poly(4-methylcyclohexyl methacrylate) | 1.4975 |
| Poly(decamethylene glycol dimethacrylate) | 1.4990 |
| Poly(vinyl alcohol) | 1.5000 |
| Poly(vinyl formal) | 1.5000 |
| Poly(2-bromo-4-trifluoromethyl styrene) | 1.5000 |
| Poly(1,2-butadiene) | 1.5000 |
| Poly(sec-butyl alpha-chloroacrylate) | 1.5000 |
| Poly(2-heptyl-1,4-butadiene) | 1.5000 |
| Poly(vinyl methyl ketone) | 1.5000 |
| Poly(ethyl alpha-chloroacrylate) | 1.5020 |
| Poly(vinyl formal) | 1.5020 |
| Poly(2-isopropyl-1,4-butadiene) | 1.5020 |
| Poly(2-methylcyclohexyl methacrylate) | 1.5028 |
| Poly(bornyl methacrylate) | 1.5059 |
| Poly(2-t-butyl-1,4-butadiene) | 1.5060 |
| Poly(ethylene glycol dimethacrylate) | 1.5063 |
| Poly(cyclohexyl methacrylate) | 1.5065 |
| Poly(cyclohexanediol-1,4-dimethacrylate) | 1.5067 |
| Butyl rubber (unvulcanized) | 1.5080 |
| Gutta percha b | 1.5090 |
| Poly(tetrahydrofurfuryl methacrylate) | 1.5096 |
| Poly(isobutylene) | 1.5100 |
| Polyethylene, low density | 1.5100 |
| Ethylene/methacrylic acid ionomer, sodium ion | 1.5100 |
| Polyethylene | 1.5100 |
| Cellulose nitrate | 1.5100 |
| Polyethylene Ionomer | 1.5100 |
| Polyacetal | 1.5100 |
| Poly(1-methylcyclohexyl methacrylate) | 1.5111 |
| Poly(2-hydroxyethyl methacrylate) | 1.5119 |
| Poly(1-butene) (isotactic) | 1.5125 |
| Poly(vinyl methacrylate) | 1.5129 |
| Poly(vinyl chloroacetate) | 1.5130 |
| Poly(N-butyl methacrylamide) | 1.5135 |
| Gutta percha a | 1.5140 |
| Poly(2-chloroethyl methacrylate) | 1.5170 |
| Poly(methyl alpha-chloroacrylate) | 1.5170 |
| Poly(2-diethylaminoethyl methacrylate) | 1.5174 |
| Poly(2-chlorocyclohexyl methacrylate) | 1.5179 |
| Poly(1,4-butadiene) (35% cis; 56% trans; 7% 1,2-content) | 1.5180 |
| Poly(acrylonitrile) | 1.5187 |
| Poly(isoprene), cis | 1.5191 |
| Poly(allyl methacrylate) | 1.5196 |
| Poly(methacrylonitrile) | 1.5200 |
| Poly(methyl isopropenyl ketone) | 1.5200 |
| Poly(butadiene-co-acrylonitrile) | 1.5200 |
| Poly(2-ethyl-2-oxazoline) | 1.5200 |
| Poly(1,4-butadiene) (high cis-type) | 1.5200 |
| Poly(N-2-methoxyethyl)methacrylamide | 1.5246 |
| Poly(2,3-dimethylbutadiene) [methyl rubber] | 1.5250 |
| Poly(2-chloro-1-(chloromethyl)ethyl methacrylate) | 1.5270 |
| Poly(1,3-dichloropropyl methacrylate) | 1.5270 |
| Poly(acrylic acid) | 1.5270 |
| Poly(N-vinyl pyrrolidone) | 1.5300 |
| Nylon 6 [Poly(caprolactam)] | 1.5300 |
| Poly(butadiene-co-styrene) (30% styrene) block copolymer | 1.5300 |
| Poly(cyclohexyl alpha-chloroacrylate) | 1.5320 |
| Poly(methyl phenyl siloxane) | 1.5330 |
| Poly(2-chloroethyl alpha-chloroacrylate) | 1.5330 |
| Poly(butadiene-co-styrene) (75/25) | 1.5350 |
| Poly(2-aminoethyl methacrylate) | 1.5370 |
| Poly(furfuryl methacrylate) | 1.5381 |
| Poly(vinyl chloride) | 1.5390 |
| Poly(butylmercaptyl methacrylate) | 1.5390 |
| Poly(1-phenyl-n-amyl methacrylate) | 1.5396 |
| Poly(N-methyl methacrylamide) | 1.5398 |
| Polyethylene, high density | 1.5400 |
| Cellulose | 1.5400 |
| Poly(cyclohexyl alpha-bromoacrylate) | 1.5420 |
| Poly(sec-butyl alpha-bromoacrylate) | 1.5420 |
| Poly(2-bromoethyl methacrylate) | 1.5426 |
| Poly(dihydroabietic acid) | 1.5440 |
| Poly(abietic acid) | 1.5460 |
| Poly(ethylmercaptyl methacrylate) | 1.5470 |
| Poly(N-allyl methacrylamide) | 1.5476 |
| Poly(1-phenylethyl methacrylate) | 1.5487 |
| Poly(2-vinyltetrahydrofuran) | 1.5500 |
| Poly(vinylfuran) | 1.5500 |
| Poly(methyl m-chlorophenylethyl siloxane) | 1.5500 |
| Poly(p-methoxybenzyl methacrylate) | 1.5520 |
| Poly(isopropyl methacrylate) | 1.5520 |
| Poly(p-isopropyl styrene) | 1.5540 |
| Poly(isoprene), chlorinated | 1.5540 |
| Poly(p,p'-xylylenyl dimethacrylate) | 1.5559 |
| Poly(cyclohexyl methyl silane) | 1.5570 |
| Poly(1-phenylallyl methacrylate) | 1.5573 |
| Poly(p-cyclohexylphenyl methacrylate) | 1.5575 |
| Poly(chloroprene) | 1.5580 |
| Poly(2-phenylethyl methacrylate) | 1.5592 |
| Hydroxypropyl cellulose | 1.5600 |
| Poly(methyl m-chlorophenyl siloxane) | 1.5600 |
| Poly[4,4-heptane bis(4-phenyl)carbonate)] | 1.5602 |
| Poly[1-(o-chlorophenyl)ethyl methacrylate)] | 1.5624 |
| Styrene/maleic anhydride copolymer | 1.5640 |
| Poly(1-phenylcyclohexyl methacrylate) | 1.5645 |
| Nylon 6,10 [Poly(hexamethylene sebacamide)] | 1.5650 |
| Nylon 6,6 [Poly(hexamethylene adipamide)] | 1.5650 |
| Nylon 6(3)T [Poly(trimethyl hexamethylene terephthalamide)] | 1.5660 |
| Poly(2,2,2'-trimethylhexamethylene terephthalamide) | 1.5660 |
| Poly(methyl alpha-bromoacrylate) | 1.5672 |
| Poly(benzyl methacrylate) | 1.5680 |
| Poly[2-(phenylsulfonyl)ethyl methacrylate] | 1.5682 |
| Poly(m-cresyl methacrylate) | 1.5683 |
| Styrene/acrylonitrile copolymer | 1.5700 |
| Poly(o-methoxyphenol methacrylate) | 1.5705 |
| Poly(phenyl methacrylate) | 1.5706 |
| Poly(o-cresyl methacrylate) | 1.5707 |
| Poly(diallyl phthalate) | 1.5720 |
| Poly(2,3-dibromopropyl methacrylate) | 1.5739 |
| Poly(2,6-dimethyl-p-phenylene oxide) | 1.5750 |
| Poly(ethylene terephthalate) | 1.5750 |
| Poly(vinyl benozoate) | 1.5775 |
| Poly[2,2-propane bis[4-(2-methylphenyl)]carbonate] | 1.5783 |
| Poly[1,1-butane bis(4-phenyl)carbonate] | 1.5792 |
| Poly(1,2-diphenylethyl methacrylate) | 1.5816 |
| Poly(o-chlorobenzyl methacrylate) | 1.5823 |
| Poly(m-nitrobenzyl methacrylate) | 1.5845 |
| Poly(oxycarbonyloxy-1,4-phenyleneisopropylidene-1,4-phenylene) | 1.5850 |
| Poly[N-(2-phenylethyl)methacrylamide] | 1.5857 |
| Poly(1,1-cyclohexane bis[4-(2,6-dichlorophenyl)]carbonate] | 1.5858 |
| Polycarbonate resin | 1.5860 |
| Bisphenol-A polycarbonate | 1.5860 |
| Makrolon® | 1.5860 |
| Lexan® | 1.5860 |
| Calibre™ | 1.5860 |
| Anjalon® | 1.5860 |
| Claritex™ | 1.5860 |
| Durolon™ | 1.5860 |
| Tarflon® | 1.5860 |
| Wonderlite® | 1.5860 |
| Airwear® | 1.5860 |
| Panlite® | 1.5860 |
| DYLEX® | 1.5860 |
| Xantar® | 1.5860 |
| MAKROCLEAR™ | 1.5860 |
| MAKROLIFE™ | 1.5860 |
| SAPHIR® | 1.5860 |
| COLORADO® | 1.5860 |
| Poly(4-methoxy-2-methylstyrene) | 1.5868 |
| Poly(o-methyl styrene) | 1.5874 |
| Polystyrene | 1.5894 |
| STYRON™ | 1.5894 |
| Poly[2,2-propane bis[4-(2-chlorophenyl)]carbonate] | 1.5900 |
| Poly[1,1-cyclohexane bis(4-phenyl)carbonate] | 1.5900 |
| Poly(o-methoxy styrene) | 1.5932 |
| Poly(diphenylmethyl methacrylate) | 1.5933 |
| Poly[1,1-ethane bis(4-phenyl)carbonate] | 1.5937 |
| Poly(propylene sulfide) | 1.5960 |
| Poly(p-bromophenyl methacrylate) | 1.5964 |
| Poly(N-benzyl methacrylamide) | 1.5965 |
| Poly(p-methoxy styrene) | 1.5967 |
| Poly(4-methoxystyrene) | 1.5967 |
| Poly[1,1-cyclopentane bis(4-phenyl)carbonate] | 1.5993 |
| Poly(vinylidene chloride) | 1.6000 |
| Poly(o-chlorodiphenylmethyl methacrylate) | 1.6040 |
| Poly[2,2-propane bis[4-(2,6-dichlorophenyl)]carbonate] | 1.6056 |
| Poly(pentachlorophenyl methacrylate) | 1.6080 |
| Poly(2-chlorostyrene) | 1.6098 |
| Poly(alpha-methylstyrene) | 1.6100 |
| Poly(phenyl alpha-bromoacrylate) | 1.6120 |
| Poly[2,2-propane bis[4-(2,6-dibromophenyl)cabonate] | 1.6147 |
| Poly(p-divinylbenzene) | 1.6150 |
| Poly(N-vinyl phthalimide) | 1.6200 |
| Poly(2,6-dichlorostyrene) | 1.6248 |
| Poly(chloro-p-xylene) | 1.6290 |
| Poly(beta-naphthyl methacrylate) | 1.6298 |
| Poly(alpha-naphthyl carbinyl methacrylate) | 1.6300 |
| Poly(phenyl methyl silane) | 1.6300 |
| Poly(sulfone) [Poly[4,4'-isopropylidene diphenoxy di(4-phenylene)sulfone]] | 1.6330 |
| Polysulfone resin | 1.6330 |
| Poly(2-vinylthiophene) | 1.6376 |
| Poly (2,6-diphenyl-1,4-phenylene oxide) | 1.6400 |
| Poly(alpha-naphthyl methacrylate) | 1.6410 |
| Poly(p-phenylene ether-sulphone) | 1.6500 |
| Poly[diphenylmethane bis(4-phenyl)carbonate] | 1.6539 |
| Poly(vinyl phenyl sulfide) | 1.6568 |
| Poly(styrene sulfide) | 1.6568 |
| Butylphenol formaldehyde resin | 1.6600 |
| Poly(p-xylylene) | 1.6690 |
| Poly(2-vinylnaphthalene) | 1.6818 |
| Poly(N-vinyl carbazole) | 1.6830 |
| Naphthalene-formaldehyde rubber | 1.6960 |
| Phenol-formaldehyde resin | 1.7000 |
| Poly(pentabromophenyl methacrylate) | 1.7100 |

**Table 2**

| |
|---|
| Coatings comprising Acrylate: e.g. Glazon® |
| Coatings comprising Organo-siloxane: e.g. SILGLIDE® |
| Coatings comprising Silane: e.g. Siliclad®, Glassclad®, Chemlon® |
| Coatings comprising Epoxy: e.g. Everslik®, Castall® |
| Coatings comprising a hydrophobic polymer |
| • Poly(tetrafluorethylene) (PTFE): e.g. Teflon™, Teflon™ -S, Teflon™-PFA, Teflon™-TFE, Teflon™-FEP, SilverStone®, SilverStone® Supra, Xylan®, Vydax®, Everlube® 72-series, Sandstrom® Poxylube®, Emralon®, Excalibur®, Ultralon®, LubriSkin™, DuraSkin™, ShieldSkin™, HardSkin™, DuraBond™ |
| • Perfluorpolyether (PFPE): e.g. Krytox® |
| • Poly(vinylidene fluoride) (PVDF): e.g. Dykor®, Kynar®, Solef® |
| • Poly(*para*-xylylene) (Parylene): e.g. Parylene AF-4, Parylene C, Parylene C-UVF®, Parylene D, Parylene HT®, Parylene N, Parylene X, ParyFree®, microRESIST™ |
| • Poly(*co*-ethylene-chlorotrifluoroethylene) (ECTFE): e.g. Halar® |
| • Poly(co-tetrafluoroethylene-hexafluoropropylene) (FEP): e.g. Precision Coating® PC 9020 |
| Coatings comprising Molybdenum disulfide (MoS₂): e.g. Ecoalube®, Perma-Slik®, Esnalube™, Lube-Lok®, Everlube® 73-series, Sandstrom® #099, Electrofilm®, Molykote®, Gun-Kote® |
| Coatings comprising MoS₂/Graphite: e.g. Lube-Lok®, Kal-Gard® |
| Coatings comprising Tungsten disulfide (WS₂): e.g. Perma-Slik® RWAC |
| Coatings comprising Graphite: e.g. Sandstrom® #75C |

**Table 3**

| Coatings comprising hydrophilic polymer/hydrogel |
|---|
| • e.g., Aculon® AcuWet coating (exact composition held as a trade secret) |
| • Poly(ethylene glycol) (PEG): e.g. Lubricent® UV, AvertPlus™ |
| • Heparin: e.g. CARMEDA® BioActive Surface |
| • N-vinyl-2-pyrrolidinone (NVP): e.g. SlipSkin™, LubriLAST™ coatings |
| • Polyurethane (PU): e.g. LubriLAST™ coatings |
| • Poly(acrylate) (PA) or Poly(methacrylate) (PMA): e.g. LubriLAST™ coatings |
| • UV/photo-active polymers: e.g. ComfortCoat® (exact composition held as a trade secret), Lubricent® UV, Serene™ hydrophilic coatings (exact composition held as a trade secret) |

### Figures

In the following, the invention is additionally illustrated in further detail by 11 figures, wherein
- **Figure 1:**: shows different designs of the device,
- **Figure 2:**: show a cross section of the device,
- **Figure 3:**: shows parts of the assembly for loading, storage and/or transport of the tissue graft or implant,
- **Figure 4:**: illustrates the process of tissue graft or implant loading into a device,
- **Figure 5:**: shows different tissue graft or implant positioning inside the device,
- **Figure 6:**: illustrates a preparation of device loaded with a tissue graft or implant aiming for direct introduction into a living body (short-term transport assembly),
- **Figure 7:**: illustrates different caps for closing the first and second opening of the device,
- **Figure 8:**: shows a possible device holder design from different perspectives, as well as a tissue culture flask with two openings,
- **Figure 9:**: illustrates the application of a macroporous material as part of the assembly for washing and/or staining of a tissue graft or implant,
- **Figure 10:**: illustrates the release of a tissue graft or implant from the device,
- **Figure 11:**: illustrates application of a 2-way extension line or a 3-way stopcock of the washing assembly for washing and/or staining of a tissue graft or implant.

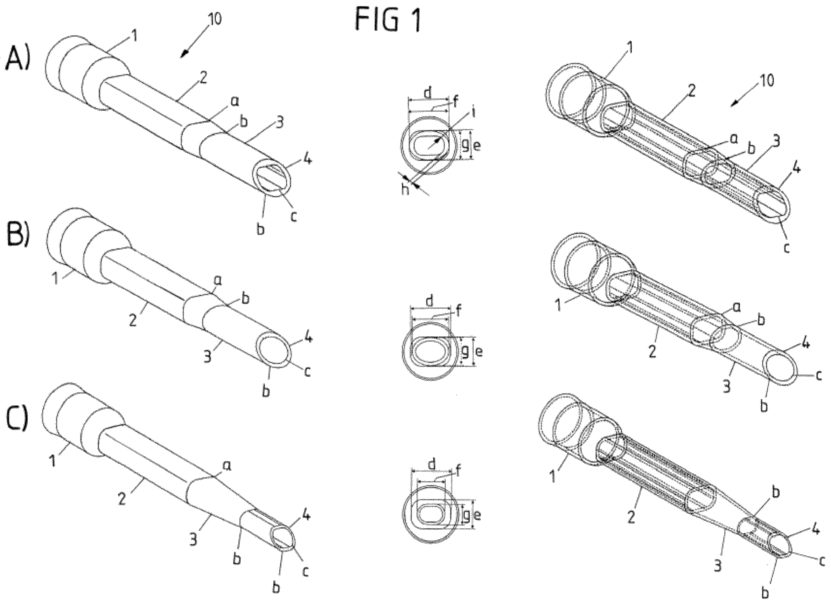

**Figure 1** illustrates three different possible designs (labelled A, Band C) of the device according to the invention. Every design is illustrated from the outside, view from the front and drawn transparently, so that the inner walls can be seen and the dimensions of some of the inner and outer compartments are illustrated. In all designs, the first opening (1), the main body (2), the taper area (3) and the second opening (4) are shown. The dimensions of the outer walls of the main body are marked with the letter (a) and are closer described by the letters (d) and (e) in the front view, the dimensions of the outer walls of the taper area are marked with the letter (b) and are closer described by the letters (f) and (g) in the front view. For all designs showed, the main body (2) has the same dimensions and shape, but can also be different according to the invention. The dimensions of the inner walls of the taper area are marked with the letter (c) and are closer described by the letter (h) and (i) in the front view. The following table gives an overview about the used markings.

| Main body (2) | |
|---|---|
| d | Outside distance between the side walls ("width") |
| e | Outside distance between the two flat and parallel opposite sides ("height") |

| Taper area (3) | |
|---|---|
| f | Outside distance between the side walls ("width") |
| g | Outside distance between the top and bottom walls ("height") |
| h | Distance between outer wall and inner wall at the smallest point |
| i | Radius of the curvature of the corners of the inner wall |

Design A shows a second opening (4) which inner wall has a rounded rectangle shape with two flat opposite walls, while the outer wall has an oval shape. The figure illustrates clearly the flat and parallel opposite sides of the main body (2), whereby the main body (2) has the following dimensions d = 3.7 mm and e = 2.7 mm. Due to the oval outer shape, the taper area (3) is slightly smaller compared to the main body (2) to fit better in a surgical incision as described above. The taper area (3) of the device (10), design A shown in Figure 1 has the following dimensions: f = 3.7 mm and g = 2.6 mm. The inner hollow compartment of the taper area (3) has rounded rectangle shape which is closer described by the following dimensions h = 0.3 mm and i = 0.7 mm.

Design B shows a second opening (4) which inner and outer wall has an oval shape. The main body (2) with its flat and parallel opposite walls has the same dimensions (d) and (e) as for the design A. The dimensions of the taper area are: f = 3.4 mm, g = 2.5 mm and h = 0.3 mm. The use of an oval shape also of the inner wall of the taper area has the advantage of a smoother tissue graft or implant ejection through the second opening of device design B, in comparison to design A.

Design C illustrates another device (10) according to the invention. The main body (2) with its flat and parallel opposite walls has the same dimensions (d) and (e) as for the design A. The taper area (3) does not have a constant thickness as design A and B and is linearly decreasing, until it remains in a constant thickness towards the second opening. At the smallest section, the taper area has the following dimensions: f = 2.5 mm, g = 1.8 mm and h = 0.3 mm and the inner walls as well as the outer walls of the taper area (3) have a rounded rectangle shape. The second opening (4) has a rounded rectangle shape as well.

All designs allow for tissue graft or implant injection in small surgical incisions (2.4 mm to 3.0 mm incision width) as described above.

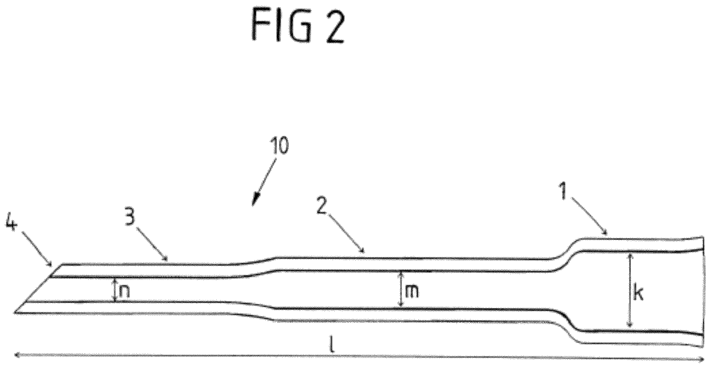

**Figure 2** shows a cross section of the device (10). Dimensions of the inner hollow compartments of the main body (2) and the taper area (3) are marked by the letters (m) and (n). Additionally, the dimension of the inner hollow compartment of the first opening (1) is marked by the letter (k). According to the invention, the first opening (1) can have a dimension (k) between 3 mm and 6 mm. The main body (2) can have a dimension (m) between 1 mm and 5 mm and the taper area (3) can have a dimension (n) between 0.8 mm and 2 mm. The overall length (I) of the device (10) is between 25 mm and 50 mm.

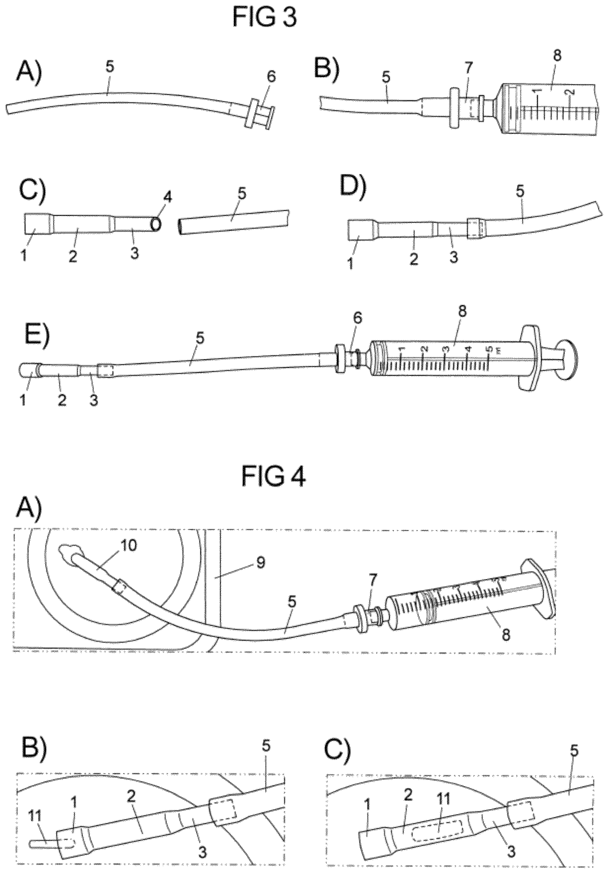

**Figures 3 (A)** to **(E)** show parts of the assembly for loading, storage and transport of a tissue graft or implant. Figure 3 (A) illustrates a tube (5) included in one embodiment of the invention with a Luer Slip connector (6). The connection of a tube (5) with a syringe (8) via a Luer Lock connector (7) is illustrated in Figure 3 (B). The second opening (4) of the device (10) is connected to the tube (5) as can be seen in Figures 3 (C) and (D). For loading a tissue graft or implant (11) into the device (10), the second opening (4) of the device (10) is connected to a tube (5) and the tube (5) is connected to a syringe (8) for example via a Luer Slip connector (6) (Figure 3 (E)).

**Figures 4(A)** to **(C)** illustrate the process of tissue graft or implant loading into a device. For loading the tissue graft or implant (11) into the device (10), a syringe (8) which is for example preferably filled with balanced salt solution or liquid nutrition medium, is connected to the second opening (4) of the device (10) by attaching one end of a tube (5) to the second opening (4) and the other end of the tube (5) to the syringe (8) via a Luer Lock connector (7). Subsequently, the syringe plunger is moved to push out excess air from the device (10) and from the tube (5), there should be no air bubbles left in the device (10) and in the tube (5) (Figure 4 (A)).

For loading the tissue graft or implant (11) into the device (10), the device (10) is attached to a laboratory dish/petri dish (9) containing the tissue graft or implant (11) in a suitable liquid medium (e.g. BSS or nutrition medium). Loading is performed from the first opening (1) which is big enough for gentle tissue graft or implant (11) uptake due to its funnel-like design (the diameter of the opening increases slightly towards the edge). Therefore, the first opening (1) of the device (10) is gently placed on top of the tissue graft or implant (11) without touching the tissue graft or implant (11), and the syringe plunger is moved to upload the tissue graft or implant (11) inside the device (10) by use of hydrodynamic flow (Figures 4 (B) and (C)).

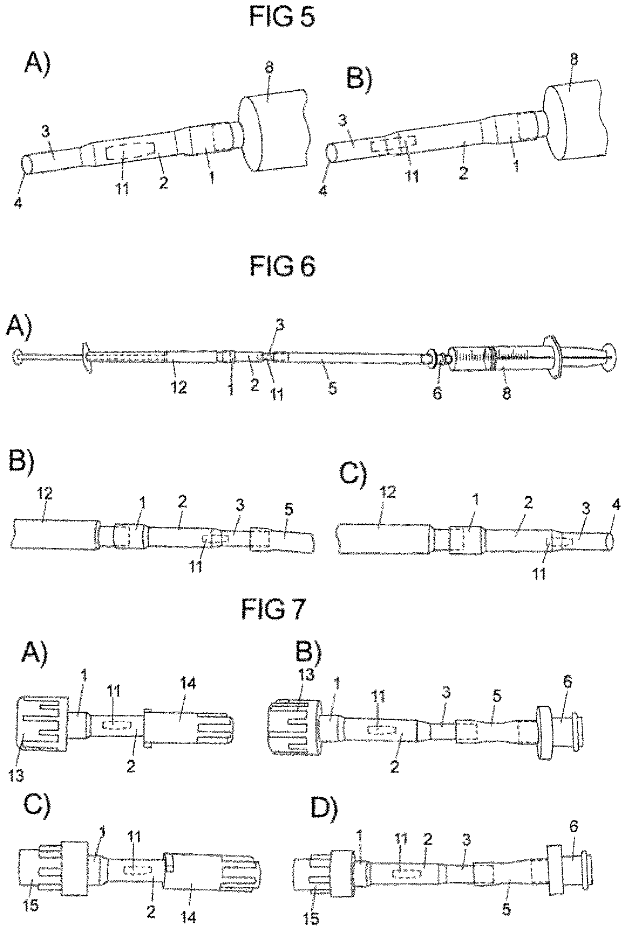

For storage and/or transport and evaluation, the tissue graft or implant (11) is positioned inside the main body (2) of the device (10), which can be called 'transport position' (Figure 4(C) and **Figure 5 (A)**)**.** For direct introduction of the tissue graft or implant (11) into for example a living body, the tissue graft or implant (11) can be positioned partially inside the taper area (3) of the device (10), which can be called 'injection position' (**Figure 5 (B)**). In this position, the tissue graft or implant (11) is partially rolled in/slightly compressed, which ensures stable positioning inside the device (10), e.g. for the attachment of a BSS-filled syringe (8 or 12) at the first opening (1) of the device (10) (**Figure 6 (A)**). The tissue graft or implant (11) could be positioned also completely inside the taper area (3) for the 'injection position'. However this increases the probability of unwanted slipping out of the tissue graft or implant (11) from the device (10) during attachment of a syringe (8 or 12) at the first opening (1) of the device (10) since in this case there is not much space left towards the second opening (4) of the device (10).

**Figures 6 (A)** to **(C)** illustrate the preparation of the device (10) loaded with a tissue graft or implant (11) aiming for direct introduction into a living body. After loading the tissue graft or implant (11) into the device (10), a second liquid-filled (e.g. balanced salt solution or nutrition medium) syringe (12) is directly connected to the first opening (1) of the device (10). At this point, the tube (5) connecting the second opening (4) and the syringe (8) used for loading the device (10) are still connected (Figure 6 (A)). After connecting the second syringe (12) to the first opening (Figure 6 (B)), the tube (5) with the syringe (8) is disconnected (Figure 6 (C)) and the device is ready for ejection of the tissue graft or implant (11), for example for injection into a living body.

According to the invention, different kind of caps are suitable to close the first (1) and the second opening (4) of the device (10). **Figures 7 (A)** to **(D)** illustrate some suitable caps. A first cap (13, 15) closes the first opening (1) and a second cap (14) closes the second opening (4) as shown in Figures 7 (A) and (C). In both embodiments, the main body (2) is left uncovered and can be used for microscopic examination and/or evaluation of the tissue graft or implant (11) by eye. At least one of the used caps is permeable to allow for exchange of oxygen and nutrients during transport and/or storage of the tissue graft or implant (11) inside the device (10). Further embodiments of the invention are shown in Figures 7 (B) and (D). The first opening (1) is still closed by a first cap (13, 15) and the second opening (4) is closed by a Luer Slip connector (6) equipped with a short flexible tubing (5), which can be of the same material as described for the tubing (5) for tissue loading (Figure 3). These embodiments have the advantage that not only the main body (2) is left uncovered but also the taper area (3), therefore both parts of the device (10) can be used for microscopic examination and/or evaluation of the tissue graft or implant (11) by eye.

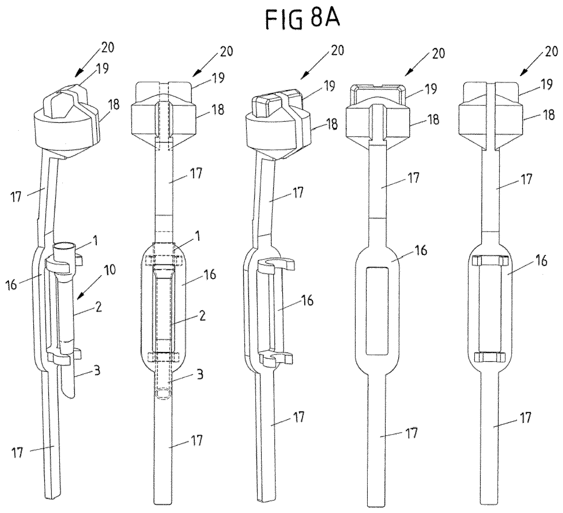

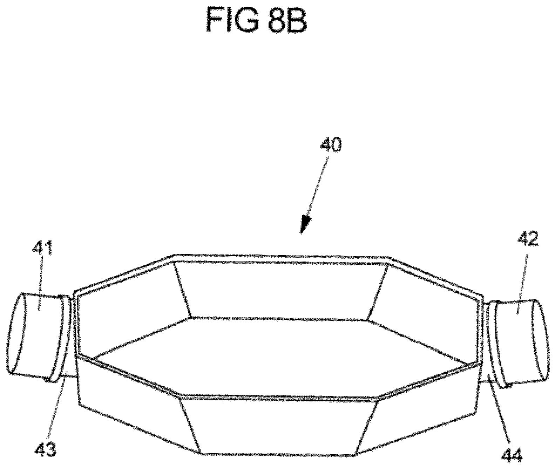

For secure storage and/or transportation of the device (10) with the tissue graft or implant (11) the present invention further provides a device holder (20) as shown in **Figure 8 (A)****.** The device holder comprises finger tips with an oval holder with a cavity (16), wherein the finger tips have cavities with a shape matching the side of the device (10) for easy assembly and gentle device (10) removal as well as for holding the device (10) tightly. Furthermore, the shape of the finger tips with an oval holder with a cavity (16) secures the orientation/position of the device (10) during storage and/or transportation. Especially, this prevents the device (10) from spinning. The finger tips with the oval holder with a cavity (16) do not interfere with the main body (2) and the taper area (3) of the device (10), which therefore can still be used as microscope examination area. The finger tips with the oval holder with a cavity (16) are attached to a tilted stripe (17) which is attached to a round neck (18) which from one side allows the insertion into a standard tissue culture flask or a tissue culture flask with two openings (40), but from another side holds tight to the neck of the neck part of the standard tissue culture flask or to one neck of the tissue culture flask with two openings (40). This prevents the movements of the whole transport device inside the standard tissue culture flask or inside the tissue culture flask with two openings (40). The round neck (18) has a holder (19) in the center which allows its manipulation with forceps and/or the end user's fingers, for secure and easy insertion and removal of the device holder (20) from the standard tissue culture flask or from the tissue culture flask with two openings (40). The overall dimensions of the device holder (20) are made in a way that the one end of the tilted stripe (17) touches the bottom of the standard tissue culture flask or the bottom of the tissue culture flask with two openings (40) while the other end with the round neck (18) is in the neck of the standard tissue culture flask or in one neck of the tissue culture flask with two openings (40) touching the closing cap of the standard tissue culture flask or one closing cap of the tissue culture flask with two openings (40) when the standard tissue culture flask or the tissue culture flask with two openings (40) is closed. **Figure 8 (B)** shows a tissue culture flask with two openings (40), suitable for insertion of the device holder (20), including the device (10). The design of the tissue culture flask with two openings (40) allows for easy aspiration of the medium (e.g. for fungal tests) directly in the device (10) from one opening (43), while the other opening (44) can be used to insert the device holder (20) including the device (10). A screw cap (41, 42) at both necks (43, 44) ensures a tight and leak-proof sealing of the tissue culture flask with two openings (40) during storage and/or transport and evaluation of the tissue graft or implant (11) being inside the device (10), which is preferably kept in place with the device holder (20) inside the tissue culture flask with two openings (40).

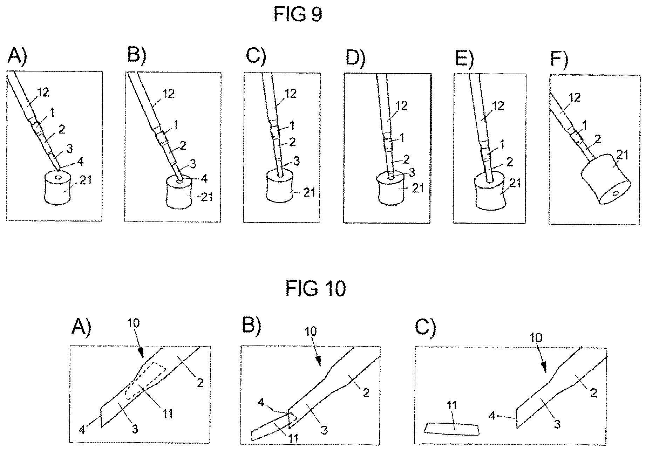

The use of a macroporous material (21) for washing and staining is shown in **Figures 9 (A)** to **(F)****.** The first opening (1) of device (10) is connected to a syringe (12) and the second opening (4) of the device (10) is positioned on top of the macroporous material (21) (Figures 9 (A) and (B)). In one embodiment, the device (10) is pressed closely against the macroporous material (21) (Figures 9 (C) and (D)), thereby providing a tight connection between macroporous material (21) and second opening (4). In another embodiment, the device (10) is pressed against the macroporous material (21) in a way that the second opening (4) as well as the taper area (3) are enclosed by the macroporous material (21) (Figures 9 (E) and (F)), therefore providing an even more tight connection. The macroporous material (21) has such dimensions that it is big enough to uptake the solution pressed out of the device (like a sponge) and that the device (10) does not break through at the bottom at macroporous material (21).

**Figures 10 (A)** to **(C)** illustrate the release of the tissue graft or implant (11) out of the device (10) by pushing a syringe (8 or 12) connected to the first opening (1). The syringe (8 or 12) is not shown. The tissue graft or implant is gently moved by a hydrodynamic flow in an oriented manner. Firstly, the tissue graft or implant (11) is positioned inside the taper area (3) of the device (10) ('injection position', Figure 10 (A)). By pushing the plunger of the syringe (8 or 12), the tissue graft or implant (11) is slipping out of the second opening (4) of the device (10) (Figure 10 (B)) and finally leaves the device (10) (Figure 10 (C)).

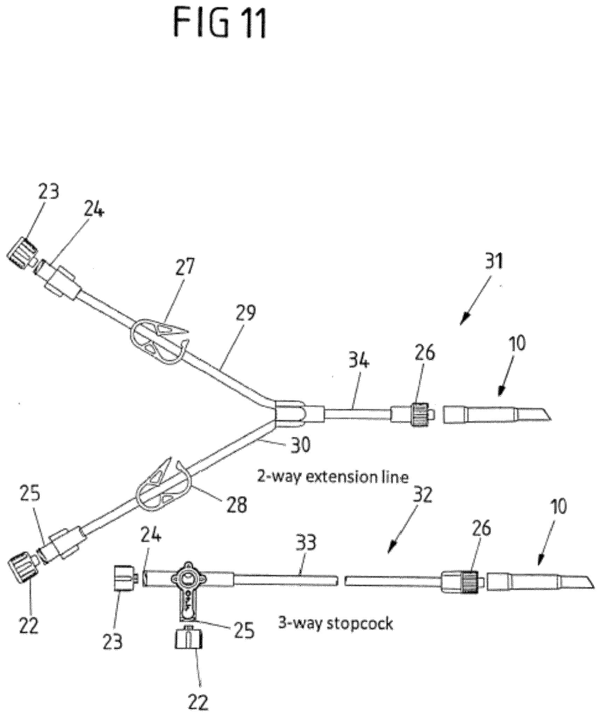

Additionally, the washing assembly comprises either a 2-way extension line (31) or 3-way stopcock (32), both comprising flexible tubes (33, 34) with male (preferably Luer Lock or Luer Slip) connectors (26), as shown in **Figure 11****.** In one embodiment of the invention, the washing assembly further comprises tube clamps (27, 28) and closing caps (22, 23) for the female endings (24, 25) of the 2-way extension line (31) or 3-way stopcock (32). The tube clamps (27, 28) can be used to interrupt the flow inside a tube (29, 30) used in the washing assembly.

The 2-way extension line (31) as well as the 3-way stopcock (32) have a flexible tube (34, 33) with a male connection (26) for attaching the device (10). The male connection (26) is preferably a Luer Lock or a Luer Slip connector. Both the 2-way extension line (31) and the 3-way stopcock (32) have two other connectors, which are preferably female connectors (24, 25). The female connectors (24, 25) are preferably Luer Lock or Luer Slip connectors. The female connectors (24, 25) are suitable to connect a conventional syringe (8) or (12) and can optionally be closed with a cap (22, 23). As shown in Figure 11, the connections with the female connectors (24, 25) are in the case of the 2-way extension line (31) equipped with tubes (29, 30), while the 3-way stopcock (32) has no tubes connected to the female connectors (24, 25). However, the availability of tubes (29, 30) is not a must.

For tissue graft or implant (11) washing and staining, a syringe (8) filled with balanced salt solution (BSS) is attached to one female connector (24) of the 2-way extension line (31) or the 3-way stopcock (32) and another syringe (12) filled with staining solution (e.g. trypan blue) is attached to the other female connector (25). The volume of the BSS-filled syringe (8) is preferably greater than the volume for the second staining solution-filled syringe (12). The syringes (8 or 12) are not shown in Figure 11.

The use of the assemblies shown in Figure 11 is described above in detail.

For removing air from the assembly, in the case of using a 2-way extension line (31), a clamp (28) of the tube (30) at the position (25) with the staining solution-filled syringe (12) remains open, while another clamp (27) at the BSS-filled syringe position (24) outlet is fully closing the tube (29). For the 3-way stopcock (32), the tap position is adjusted in a way that the staining solution-filled syringe (12) at position (25) and the male connection (26) outlet are connected while the position (24) with the BSS-filled syringe (8) remains closed.

First, the tubing (30) and the position (25) attached to the staining solution-filled syringe (12) is fully flushed with staining solution. The staining solution must not enter the male connection (26) of the assembly (31, 32), since this step is only needed for air removal. Next, complete air removal and flush of the assembly with BSS solution needs to be performed. In the case of using a 2-way extension line (31), a clamp (28) of the tube (30) at the position (25) with the staining solution-filled syringe (12) is fully closing the tube (30), while another clamp (27) of the tube (29) at the BSS-filled syringe (8) position (24) outlet remains open. For the 3-way stopcock (32), the tap position is adjusted in a way that the BSS-filled syringe (8) at position (24) and the male connection (26) outlet are connected while the position (25) with the staining solution-filled syringe (12) remains closed.

Now, the assembly is fully flushed with BSS by pushing the plunger of the BSS-filled syringe (8). There should be no air left in the assembly as well as there should be no staining solution eluting from the male connection of the assembly. Fourth, the device (10) containing the tissue graft or implant (11) is attached via the first opening (1) to the male connector (26) of the 2-way extension line (31) or 3-way stopcock (32) and placed into a BSS-filled laboratory dish/petri dish (9, not shown in Figure 11) to prevent air infiltration to the device (10).

To decrease the probability of tissue graft or implant (11) slipping out of the device (10), the second opening (4) of the device (10) can be closed by a cap (13, 14, 15) or can be placed onto/into the macroporous material (21) from the washing assembly during the whole staining and washing process. If applicable, the liquid transport medium can now be flushed out of the device (10) prior staining by gently pushing the plunger of the BSS-filled syringe (8) at position (24). Alternatively, staining of the tissue graft or implant (11) can be performed without prior washing with BSS solution.

Next, for the use of a 2-way extension line (31), the clamp (27) at the BSS-filled syringe (8) position (24) is fully closing the tube (29), while the other clamp (28) of the tube (30) at the position (25) with the staining solution-filled syringe (12) outlet remains open. In case of the use of a 3-way stopcock (32), the tap position is adjusted in a way that the staining solution-filled syringe (12) at position (25) and the male connection (26) outlet are connected while the position (24) with the BSS-filled syringe (8) remains closed.

Now the staining solution is gently added to the tissue graft or implant (11) in the device (10) by pushing the plunger of the staining solution-filled syringe (12) at position (25) and left for at least 1-2 minutes for sufficient staining. Extended staining will cause increased cell damage. After staining, in the case of using a 2-way extension line (31), the clamp (28) at the position (25) with the staining solution-filled syringe (12) is fully closing the tube (30), while the other clamp (27) of the tube (29) at the BSS-filled syringe (8) position (24) outlet remains open. For the 3-way stopcock (32), the tap position is adjusted in a way that the BSS-filled syringe (8) at position (24) and the male connection (26) outlet are connected while the position (25) with the staining solution-filled syringe (12) remains closed.

Lastly, the device (10) is fully flushed with BSS by pushing the plunger of the BSS-filled syringe (8). The solution in the device (10) should be completely transparent and no staining solution should remain. Now, the device (10) and the respective tissue graft or implant (11) are ready for ejection of the tissue graft or implant (11), especially for injection the tissue graft or implant (11) into the living body.

### List of reference numerals

- 1: First opening
- 2: Main body
- 3: Taper area
- 4: Second opening
- 5: Tube
- 6: Luer Slip connector
- 7: Luer Lock connector
- 8: Syringe
- 9: Laboratory dish/petri dish
- 10: Device
- 11: Tissue graft or implant
- 12: Syringe
- 13: Cap
- 14: Cap
- 15: Cap
- 16: Finger tips with oval shaped platform with a cavity
- 17: Tilted stripe
- 18: Round neck
- 19: Holder
- 20: Device holder
- 21: Macroporous material
- 22: Cap
- 23: Cap
- 24: Female connector
- 25: Female connector
- 26: Male connector
- 27: Tube clamp
- 28: Tube clamp
- 29: Tube
- 30: Tube
- 31: 2-way extension line
- 32: 3-way stopcock
- 33: Tube
- 34: Tube
- 40: Tissue culture flask with two openings
- 41: Screw Cap
- 42: Screw Cap
- 43: Neck with thread
- 44: Neck with thread

### References

- [1]: Williams K A, et al. Transplantation. 2006;81:896-901
- [2]: Price M O & Price F W, Clin Exp Ophthalmol. 2010;38,128-140
- [3]: Hamzaoglu E C, et al. Ophthalmology. 2015 122;11,2193-2199
- [4]: Uchino Y, et al. Cornea. 2011;30:287-290
- [5]: Li S, et al. PLoS ONE. 2017;12(12): e0182275
- [6]: Melles G R, et al. Cornea. 2002;21:415-418
- [7]: Melles G R, et al. Cornea. 2006;25:987-990
- [8]: Anshu A, et al. Ophthalmology. 2012;119:536-540
- [9]: Tourtas T, et al. Am J Ophthalmol. 2012;153:1082-1090
- [10]: Guerra F P, et al. Ophthalmology. 2011;118:2368-2373
- [11]: Guerra F P, et al. Cornea. 2011;30:1382-1386
- [12]: Ham L, et al. Arch Ophthalmol. 2009;127:252-255
- [13]: 57th annual Eye Bank Association of America meeting Philadelphia, USA 2018; personal discussions with Dr. Pankaj Gupta, University Hospitals of Cleveland, USA
- [14]: Busin M & Albé E Curr Opin Ophthalmol. 2014;25:312-318
- [15]: Groeneveld-van Beek, et al. Acta Ophthalmol. 2013;91:145-150
- [16]: Baydoun L, et al. Am J Ophthalmol. 2012;154:762-763
- [17]: Dapena I, et al. Arch Ophthalmol. 2011;129:88-94
- [18]: Eye Bank Association of America, Eye Banking Statistical Report 2017
- [19]: Eye Bank Association of America, Medical Standards 2015
- [20]: OR visit in Eye Clinic Chemnitz, Germany 2018; *personal discussions with MD Roy Schendel,* Klinikum Chemnitz, Germany
- [21]: Kobayashi A, et al. BMC Ophthalmol. 2016;16:135
- [22]: Park C Y, et al. Ophthalmology. 2015;122:2432-2442
- [23]: Deutsche Gesellschaft für Gewebetransplantation Hannover, Germany 2015; "LaMEK" tissue (pre-prepared DMEK grafts)
- [24]: Lions VisionGift Portland, USA 2018; "Patient Ready DMEK™"
- [25]: Tran K D, et al. Cornea. 2017;36:484-490
- [26]: Parekh M, et al. Am J Ophthalmol. 2016;166:120-125
- [27]: Augenklinik Sulzbach, Germany and Geuder AG Heidelberg, Germany 2016; "Preloaded DMEK"/"Vorgeladenes Glaskartuschen-Mikroinjektorsystem"
- [28]: 57th annual Eye Bank Association of America meeting Philadelphia, USA 2018; Technical Skills Workshop
- [29]: Parekh M, et al. Acta Ophthalmol. 2017;95:194-198
- [30]: XXXIst annual European Eye Bank Association meeting Rotterdam, The Netherlands 2019; Lamellar graft Session VI
- [31]: Lee C H, et al. J. Micromech. Microeng. 2010;20:035018
- [32]: Lee S M, et al. J. Micromech. Microeng. 2008;18:125007
- [33]: Annabi N, et al. Tissue Eng. Part B. Rev. 2010;16:371-383
- [34]: Hollister S J Nat. Mater. 2005;4:518-524
- [35]: Mikos A G & Temenoff J S Electron. J. Biotechnol. 2000;3:114-119
- [36]: Chiu Y-C, et al. Tissue Eng. Part C. Methods. 2010;16:905-912
- [37]: Murphy W L, et al. Tissue Eng. 2002;8:43-52
- [38]: Sannino A, et al J. Biomed. Mater. Res. A. 2006;79:229-236
- [39]: Ma P X & Choi J W Tissue Eng. 2001;7:23-33
- [40]: Dziubla T D PhD Thesis. 2002
- [41]: Levesque S G, et al. Biomaterials 2005;26:7436-7446
- [42]: Pham Q P, et al. Tissue Eng. 2006;12:1197-1211
- [43]: Lozinsky V I Russ. Chem. Rev. 2007;71:489-511
- [44]: Kaetsu I Adv. Polym. Sci. 2005;105:81-97
- [45]: Hassan C & Peppas N Adv. Polymer Sci. 2000;153:37-65

## Claims

1. Device (10) for secure support storage and/or transport of a tissue graft or implant (11), comprising:
• a first opening (1),
• a main body (2),
• a taper area (3), and
• a second opening (4),
wherein the first opening (1) has a round shape and a funnel-like design configured to connect with a tube (5) or a syringe (8, 12) and the main body (2) is transparent and has a rectangle shape and the taper area (3) is transparent and has a an elliptical, round, biconvex or rectangle shape, and wherein the main body (2) comprises at least two flat and parallel opposite sites.

2. Device (10) according to claim 1, wherein the second opening (4) has an elliptical, round, biconvex or rounded rectangle shape.

3. Device (10) according to any one of claims 1 to 2, wherein the outer wall of the second opening (4) has a different shape than the inner wall.

4. Device (10) according to any one of claims 1 to 3, wherein the outer wall of the taper area (3) has a different shape than the inner wall.

5. Device (10) according to any one of claims 1 to 4, wherein the device (10) consists of glass, preferably borate glass or consists of plastic, wherein said plastic is transparent and has a refractive index (rᵢ) in the range of rᵢ = 1.30 to rᵢ = 1.71, preferably rᵢ = 1.30 to rᵢ = 1.65, most preferably 1.30 to rᵢ = 1.60, which is similar to the rᵢ = 1.33 to 1.34 (at 20 °C and 600 nm) of balanced salt solution (BSS), such as Hank's BSS, Earle's BSS, Tyrode's salt solution, Alsever's salt solution, phosphate-buffered saline (PBS), Tris-buffered saline (TBS), Puck's salt solution, Gey's salt solution, Ringer's salt solution, Simm's salt solution and related buffered saline solutions.

6. Device (10) according to claim 5, wherein the inner surface of the device (10) comprises
• a hydrophobic coating, selected from coatings comprising acrylate, organo-siloxane, silane, epoxy, a polymer, Molybdenum disulfide, Molybdenum disulfide/graphite, Tungsten disulfide or graphite, preferably selected from coatings comprising a polymer, organo-siloxane, silane, acrylate or epoxy; or
• a hydrophilic coating, selected from coatings comprising any hydrophilic polymer/hydrogel, preferably selected from coatings comprising poly(ethylene glycol), poly(acrylate), poly(methacrylate) or a UV/photo-active polymer; and/or
• a surface patterning, being a micro- or nanostructured surface pattern or a combination thereof (e.g. nano-microstructuring) in the range of 100 nm to 20000 nm, preferably between 300 nm to 5000 nm and more preferably between 500 nm to 2500 nm.

7. Device (10) according to any one of claims 1 to 6, further comprising a cap (13, 14, 15) for the first opening (1) and/or a cap (13, 14, 15) for the second opening (4).

8. Assembly for loading, storage and transport of a tissue graft or implant (11), comprising
• the device (10) according to claims 1 to 7,
• at least one syringe (8, 12),
• a tube (5), and
• at least one cap (13, 14, 15).

9. Assembly according to claim 8, further comprising a transport device.

10. Assembly according to claim 9, wherein the transport device is a device holder (20) comprising
• a round neck (18) with a holder (19),
• finger tips which are attached on an oval shaped platform with a cavity (16), and
• a tilted stripe (17),
which is suitable to be positioned inside a container, such as a standard tissue culture flask or a tissue culture flask with two openings (40).

11. Assembly according to claim 10, wherein the finger tips and the oval shaped platform with a cavity (16) do not optically interfere with the main body (2) and the taper area (3) of the device (10).

12. Washing assembly for washing and staining a tissue graft or implant (11), comprising
• a macroporous material (21),
• the device (10) according to claims 1 to 7 or comparable conventional devices,
• at least one syringe (8, 12),
• optionally at least one cap (13, 14, 15, 22, 23),
• optionally at least one tube clamp (27, 28), and
• a 2-way extension line (31) or a 3-way stopcock (32), wherein both optionally comprise flexible tubes (29, 30, 33, 34).

13. Method for preparing a tissue graft or implant (11) using the device (10) according to claims 1 to 7, comprising the steps:
a) providing a tissue graft or implant (11),
b) loading the tissue graft or implant (11) into the device (10),
c) sealing the device (10) by at least one cap (13, 14, 15),
d) evaluation and quality control of the tissue graft or implant (11) inside the device (10),
e) transport of the device (10) with the tissue graft or implant (11),
f) optionally evaluation and quality control of the tissue graft or implant (11) inside the device (10),
g) washing and staining of the tissue graft or implant (11) inside the device (10).

14. Method according to claim 13, wherein the device (10) is transported within the device holder (20).

15. Method according to any one of claims 13 to 14, wherein evaluation and quality control of the tissue graft or implant (11) is performed with the device (10) containing the tissue graft or implant (11) being inside the transport device and inside a container, such as a standard tissue culture flask or a tissue culture flask with two openings (40).
